# DEMANDE DE BREVET EUROPEEN

(11) **EP 1 935 402 A1**
(43) Date de publication de la demande: **25.06.2008**
(21) Numéro de dépôt: 07123594.9
(22) Date de dépôt: 19.12.2007
(51) Int. Cl.: A61K 8/89, A61Q 1/00, A61Q 5/00, A61Q 19/00

(54) **Emulsion cosmétique contenant des composés siliconés**

(30) Priorité: 20.12.2006 FR 0655705
(71) Demandeur: L'ORÉAL, 75008 Paris (FR)
(72) Inventeur: Cassin, Guillaume, 91140 Villebon Sur Yvette (FR)
(74) Mandataire: Le Coupanec, Pascale A.M.P.

(57) **Abrégé**

La présente invention a pour objet principalement un kit cosmétique de maquillage ou de soin non thérapeutique de matière(s) kératinique(s) comprenant au moins deux compositions et contenant au moins un composé X et au moins un composé Y, les composés X et Y étant aptes à réagir ensemble, le cas échéant en présence d'un catalyseur, et l'un au moins des composés étant siliconé, avec l'une au moins des compositions étant une émulsion.

## Description

La présente invention a pour objet principalement un kit cosmétique de maquillage ou de soin non thérapeutique de matière(s) kératinique(s) comprenant au moins deux compositions et contenant au moins un composé X et au moins un composé Y, les composés X et Y étant aptes à réagir ensemble, le cas échéant en présence d'un catalyseur ou d'un peroxyde, et l'un au moins des composés étant siliconé, avec l'une au moins des compositions étant une émulsion.

Les kits selon l'invention peuvent être des produits de maquillage ou de soin des matières kératiniques, en particulier de la peau, des lèvres, des cils, des sourcils ou des ongles.

Plus précisément les produits de maquillage peuvent être de type fonds de teint, fards à joues ou à paupières, produits anti-ceme, blush, rouges à lèvres, baumes à lèvres, brillants à lèvres, mascaras, eye-liner ou encore un produit de maquillage du corps ou de coloration de la peau.

Les produits de soin de la peau peuvent être une composition de protection, de traitement ou de soin pour le visage, pour les mains, pour les pieds, pour les grands plis anatomiques ou pour le corps (par exemple crèmes de jour, crème de nuit, crème démaquillante, composition anti-solaire, laits corporels de protection ou de soin, laits après-solaire, lotion, gel ou mousse pour le soin de la peau, composition de bronzage artificiel); une composition après-rasage.

La présente invention vise plus particulièrement à proposer un nouveau mode de formulation de compositions cosmétiques, permettant d'obtenir un film déposé sur les matières kératiniques doté de bonnes propriétés cosmétiques notamment de tenue, de matification et formant un dépôt confortable sur la peau.

Récemment, les inventeurs ont constaté qu'il est possible d'obtenir de telles propriétés en mettant à profit la faculté de certains composés, notamment siliconés, à interagir lorsqu'ils sont mis en présence, le cas échéant en présence d'un catalyseur ou d'un peroxyde, et à constituer à l'issue de leur interaction un film polymérique.

Ainsi des composés dits composé X et composé Y, tels que définis ci-après, s'avèrent capables de polymériser *in situ,* à pression atmosphérique et température ambiante et de former des films avantageusement biocompatibles, non collants, légèrement opalescents voire pelables. De tels systèmes sont notamment en partie décrits dans les brevets WO 01/96 450 et GB 2 407 496 de Dow Corning.

Ces films polymériques, susceptibles d'être formés *in situ* sur un support, notamment de type matière kératinique, s'avèrent dotés de propriétés avantageuses en terme de cosmétique, à savoir bonne adhésion, bonne tenue et confort.

D'une manière générale, ces films sont également dotés d'un certain degré de brillance.

Or, si ces propriétés de brillance sont recherchées pour certains produits cosmétiques à l'image des rouges à lèvres et des vernis à ongles, elles peuvent en revanche s'avérer indésirables pour d'autres produits cosmétiques à l'image des fonds de teint et des produits de soin. En conséquence, la présente invention vise précisément à proposer des produits cosmétiques de maquillage et/ou de soin aptes à former un film *in situ* par mise en contact des composés précités et qui soient en revanche dénués d'effet brillant.

Les inventeurs ont découvert qu'il était possible d'obtenir les propriétés précitées sous réserve de formuler au moins l'un des composés X et Y dans la phase huileuse d'une émulsion simple ou multiple E/H/E.

On entend par émulsion simple une émulsion huile-dans-eau (H/E) ou eau-dans-huile (E/H).

Ainsi, selon un premier aspect, la présente invention a pour objet un kit cosmétique de maquillage ou de soin non thérapeutique de matière(s) kératiniques(s) comprenant au moins deux compositions différentes conditionnées séparément, le kit comprenant au moins un composé X, au moins un composé Y, et éventuellement au moins un catalyseur ou un peroxyde, avec au moins un des composés X ou Y étant siliconé et lesdits composés X et Y étant susceptibles de réagir ensemble par une réaction d'hydrosilylation en présence d'un catalyseur, ou par une réaction de condensation, ou par une réaction de réticulation en présence d'un peroxyde, lorsqu'ils sont mis en contact les uns avec les autres, ledit kit étant tel que les composés X et Y, et le catalyseur ou le peroxyde lorsqu'ils sont présents, ne sont pas présents simultanément dans la même composition et qu'au moins l'une des compositions dudit kit est sous la forme d'une émulsion simple ou multiple E/H/E dans laquelle ledit composé X ou Y est présent dans la phase huileuse.

Le ou les composés X et le ou les composés Y, peuvent être appliqués sur les matières kératiniques à partir de plusieurs compositions contenant le ou les composés X et le ou les composés Y, seuls ou en mélange, ou à partir d'une seule composition contenant le ou les composés X et le ou les composés Y.

Selon une première variante de réalisation, ledit kit comprend au moins
i. une première composition contenant dans un milieu physiologiquement acceptable au moins un composé X et
ii. une seconde composition contenant, dans un milieu physiologiquement acceptable, au moins un composé Y,
avec au moins l'une desdites première et seconde compositions étant sous la forme d'une émulsion simple ou multiple E/H/E dans laquelle le composé X ou Y est présent en phase huileuse, l'une au moins desdites première et seconde compositions contenant, en outre, le cas échéant, au moins un catalyseur ou un peroxyde.

Les première et seconde compositions sont différentes l'une de l'autre.

Par exemple, la première composition est avantageusement dénuée de composé Y et la seconde composition est avantageusement dénuée de composé X. En effet, au regard de leur grande réactivité l'un pour l'autre, les composés X et Y ne sont pas présents simultanément dans une première et/ou seconde composition formant un kit selon l'invention lorsque leur interaction n'est pas conditionnée par la présence d'un catalyseur ou d'un peroxyde.

Selon un premier mode de réalisation, ledit kit peut contenir une seule des deux compositions sous la forme d'une émulsion.

Selon un second mode de réalisation les deux compositions sont sous la forme d'une émulsion, de type simple ou multiple E/H/E. Selon une seconde variante de réalisation, ledit kit comprend au moins
i. une première composition contenant dans un milieu physiologiquement acceptable au moins un composé X et un composé Y, lesdits composés X et Y étant susceptibles de réagir ensemble en présence d'un catalyseur par une réaction d'hydrosilylation, ou par une réaction de condensation, ou par une réaction de réticulation en présence d'un peroxyde, lorsqu'ils sont mis en contact l'un avec l'autre, et
ii. une seconde composition contenant, dans un milieu physiologiquement acceptable, au moins ledit catalyseur ou ledit peroxyde nécessaire à l'interaction des composés X et Y,
avec ladite première composition étant sous la forme d'une émulsion simple ou multiple (E/H/E) dans laquelle les composés X et Y sont présents en phase huileuse.

En particulier, le kit selon l'invention peut comprendre un catalyseur.

En particulier, ledit catalyseur est formulé en phase aqueuse.

Avantageusement, la ou les compositions sous la forme d'une émulsion dans les kits selon l'invention sont des émulsions directes huile dans eau.

De manière inattendue, le film obtenu à l'issue de l'application des compositions formant le kit selon l'invention s'avère dénué d'effet brillant.

Ainsi, ce film peut être caractérisé par un paramètre matifiant figuré par le symbole R, dont la valeur est inférieure à 1,2 lorsqu'elle est déterminée selon le protocole décrit ci-après.

De préférence, les compositions du kit et en particulier la première composition comprenant le composé X et la seconde composition comprenant le composé Y du kit sont conditionnées dans des conditionnements séparés.

Par exemple, chaque composition peut être conditionnée séparément dans un même article de conditionnement par exemple dans un stylo bi-compartimenté, la composition de base étant délivrée par une extrémité du stylo et la composition du dessus étant délivrée par l'autre extrémité du stylo, chaque extrémité étant fermée notamment de façon étanche par un capuchon. Chaque composition peut aussi être conditionnée dans un compartiment au sein d'un même article conditionnement, le mélange des deux compositions s'effectuant à la ou les extrémités de l'article de conditionnement lors de la délivrance de chaque composition.

Alternativement, chacune des première et seconde compositions peut être conditionnée dans un article de conditionnement différent.

Au sens de l'invention, il est entendu que le mélange formé, quel que soit le conditionnement, comprend des composés X et/ou Y sous une forme n'ayant pas encore réagi et non pas exclusivement sous la forme de leur produit de réaction par hydrosilylation en présence d'un catalyseur, par condensation et/ou par réticulation en présence d'un peroxyde.

Ainsi, la formation du produit de réaction selon l'invention, peut être soit réalisée directement sur la surface de la matière kératinique devant être traitée, soit initiée juste avant application par mélange extemporané des composés X et Y dans des conditions propices à leur interaction, la formation du produit de réaction étant dans ce dernier cas finalisée en surface de la matière kératinique.

Pour des raisons évidentes, et au regard de la grande réactivité des composés X et/ou Y, il est en effet nécessaire que leur mise en oeuvre soit réalisée dans des conditions propices à la maniabilité de la composition le (ou les) contenant notamment en vue de son étalement par exemple. Le procédé conforme à l'invention met donc en oeuvre une composition contenant des composés X et Y, et donc non figée sous la forme du film final attendu et résultant de la réaction de la totalité de X et/ou de la totalité de Y.

L'invention concerne également un procédé de soin cosmétique et/ou de maquillage de matière(s) kératinique(s) comprenant au moins l'application sous la forme d'au moins une émulsion simple ou multiple (E/H/E) (a) d'un ou plusieurs composés X, (b) d'un ou plusieurs composés Y avec au moins un des composés X et Y étant siliconé et lesdits composés X et Y étant susceptibles de réagir ensemble par une réaction d'hydrosylilation en présence d'un catalyseur, ou par une réaction de condensation, ou par une réaction de réticulation en présence d'un peroxyde, lorsqu'ils sont mis en contact l'un avec l'autre, et le cas échéant, (c) d'un catalyseur ou d'un peroxyde, si nécessaire à l'interaction dudit composé X avec ledit composé Y, les applications (a), (b) et (c) pouvant être simultanées ou séquencées selon un ordre indifférent sous réserve qu'il soit propice à l'interaction desdits composés X et Y.

Ainsi, le ou les composés X, le ou les composés Y, peuvent être appliqués sur les matières kératiniques à partir de plusieurs compositions, les compositions contenant respectivement le ou les composés X, le ou les composés Y et le cas échéant un catalyseur ou un peroxyde, seuls ou en mélange, ou à partir d'une seule composition contenant le ou les composés X et le ou les composés Y et le cas échéant un catalyseur ou un peroxyde.

Selon un mode de réalisation particulier, on applique, selon un procédé cosmétique de maquillage et/ou de soin des matières kératiniques, notamment de la peau humaine, sur lesdites matières kératiniques, au moins une couche d'une première composition contenant dans un milieu physiologiquement acceptable au moins un composé X, et au moins une couche d'une seconde composition contenant dans un milieu physiologiquement acceptable au moins un composé Y, avec l'une au moins desdites première et seconde compositions étant sous la forme d'une émulsion simple ou multiple (E/H/E) contenant le composé X ou Y dans sa phase huileuse, et l'une au moins des première et seconde compositions contenant en outre, le cas échéant, au moins un catalyseur ou un peroxyde.

On peut également appliquer en alternance sur les matières kératiniques plusieurs couches de chacune des première et seconde compositions.

Plus particulièrement, ledit procédé peut consister à appliquer sur lesdites matières kératiniques au moins une composition sous la forme d'une émulsion simple ou multiple (E/H/E) comprenant, dans sa phase huileuse, au moins un composé X et au moins un composé Y, ladite composition contenant, en outre, le cas échéant, au moins un catalyseur ou un peroxyde.

La composition appliquée peut être également obtenue en mélangeant de façon extemporanée une première composition contenant au moins le composé X et une seconde composition contenant au moins le composé Y, l'une au moins des première et seconde compositions étant sous la forme d'au moins une émulsion simple ou multiple (E/H/E), et contenant, en outre, le cas échéant, au moins un catalyseur ou un peroxyde.

Comme précisé ci-dessus, les compositions sont avantageusement sous la forme d'une émulsion simple ou multiple E/H/E.

Selon un mode de réalisation, au moins une couche supplémentaire d'au moins une troisième composition comprenant un milieu cosmétiquement acceptable, et de préférence au moins un polymère filmogène et au moins un milieu solvant organique (ou huileux) ou aqueux, est appliquée sur la ou les couches de la ou les compositions selon l'invention comprenant les composés X, Y, et, le cas échéant, un catalyseur ou un peroxyde, afin par exemple d'améliorer la tenue et/ou le confort, de celle(s)-ci.

Selon un second aspect, la composition mise en oeuvre dans le procédé de l'invention peut être une composition unique sous forme d'une émulsion simple ou multiple (E/H/E) et contenant les composés X, Y et, le cas échéant, un peroxyde ou un catalyseur en phase huileuse, avec au moins l'un des composés X et Y étant sous une forme encapsulée.

Ainsi, la présente invention vise également une composition cosmétique sous forme d'émulsion simple ou multiple (E/H/E) notamment de soin et/ou de maquillage de matière(s) kératinique(s) contenant dans un milieu physiologiquement acceptable au moins un composé X en phase huileuse, un composé Y en phase huileuse tels que définis ci-dessus et, le cas échéant, un catalyseur ou un peroxyde, avec au moins un des composés X et Y étant sous une forme encapsulée.

Selon une variante de réalisation préférée, les deux composés X et Y sont présents sous des formes encapsulées séparées.

Selon ce mode de réalisation, les deux composés X et Y peuvent être conditionnés dans une même composition tout en s'affranchissant du risque de réaction prématurée entre eux. Cette réaction n'intervient qu'au moment où la composition est manipulée préalablement ou au moment de son application sur la matière kératinique. La ou les forme(s) encapsulée(s) et les composés X et Y peuvent réagir pour former le film attendu.

### DETERMINATION DU CARACTERE MATIFIANT

Le caractère matifiant quantifié par la valeur R est caractérisé par une mesure au gonioréflectomètre de la société Micromodule, équipé d'un bras articulé de la société Newport ESP 300.

Pour ce faire, on étale un mélange obtenu à partir de composition(s) conforme(s) à l'invention, en particulier de première et seconde compositions selon l'invention, en proportion 50/50 sur une carte de contraste (Prufkarte type 24/5 - 250 cm² commercialisée par la société Erichsen) à l'aide d'un tire-film mécanique (épaisseur humide 150 microns).

On sèche ensuite la composition ainsi obtenue pendant une nuit à une température de 37 °C, puis on mesure la réflexion à l'aide d'un gonioréflectomètre.

Le résultat obtenu est le rapport R entre la réflexion spéculaire et la réflexion diffuse. La valeur de R est d'autant plus faible que l'effet matifiant est important.

Avantageusement ce film peut être caractérisé par un paramètre matifiant R, de valeur inférieure à 1,2, en particulier inférieure à 1, notamment inférieure à 0,8 voire inférieure à 0,75.

### COMPOSES X ET Y

Par composé siliconé, on entend un composé polyorganosiloxane, c'est-à-dire comprenant au moins deux unités organosiloxanes, par exemple au moins 5 unités organosiloxane, notamment au moins 10 unités organosiloxane. Selon un mode de réalisation particulier, l'un au moins des composés X et Y, ou les composés X et les composés Y sont siliconés. Les composés X et Y peuvent être aminés ou non aminés.

Selon un autre mode de réalisation, au moins un des composés X et Y est un polymère dont la chaîne principale est formée majoritairement d'unités organosiloxanes. Parmi les composés siliconés cités ci-après, certains peuvent présenter à la fois des propriétés filmogènes et adhésives, selon par exemple leur proportion en silicone ou selon qu'on les utilise en mélange avec un additif particulier. Il est par conséquent possible de moduler les propriétés filmogènes ou les propriétés adhésives de tels composés selon l'utilisation envisagée, c'est en particulier le cas pour les silicones élastomères réactives dites " room temperature vulcanization".

Les composés X et Y peuvent réagir ensemble à une température variant entre la température ambiante et 180°C. Avantageusement les composés X et Y sont susceptibles de réagir ensemble à température ambiante (20 ± 5°C) et pression atmosphérique, ou avantageusement en présence d'un catalyseur, par une réaction d'hydrosilylation ou une réaction de condensation, ou une réaction de réticulation en présence d'un peroxyde.

### Groupes polaires

Selon un mode de réalisation particulier, l'un au moins des composé X et Y, par exemple le composé X, est porteur d'au moins un groupe polaire susceptible de former au moins une liaison hydrogène avec les matières kératiniques.

Par groupe polaire, on entend un groupe comportant des atomes de carbone et d'hydrogène dans sa structure chimique et au moins un hétéroatome (tel que O, N, S et P), tel que ledit groupe est apte à établir au moins une liaison hydrogène avec les matières kératiniques.

Des composés porteurs d'au moins un groupement apte à établir une liaison hydrogène sont particulièrement avantageux, car ils apportent aux compositions les contenant une meilleure adhérence sur les matières kératiniques.

Le ou les groupe(s) polaire(s) porté(s) par au moins l'un des composés X et Y est/sont apte(s) à établir une liaison hydrogène, et comporte(nt) soit un atome d'hydrogène lié à un atome électronégatif, soit un atome électronégatif comme par exemple l'atome d'oxygène, d'azote ou de soufre. Lorsque le groupe comporte un atome d'hydrogène lié à un atome électronégatif, l'atome d'hydrogène peut interagir avec un autre atome électronégatif porté, par exemple par une autre molécule, telle que la kératine, pour former une liaison hydrogène. Lorsque le groupement comporte un atome électronégatif, l'atome électronégatif peut interagir avec un atome d'hydrogène lié à un atome électronégatif porté, par exemple par une autre molécule, telle que la kératine, pour former une liaison hydrogène.

Avantageusement, ces groupes polaires peuvent être choisis parmi les groupes suivants :
- acides carboxyliques -COOH,
- alcools, tels que : -CH₂OH ou -CH(R)OH, R étant un radial alkyle comprenant de 1 à 6 atomes de carbone,
- amino de formule -NR₁R₂, dans laquelle les R₁ et R₂ identiques ou différents représentent un radial alkyle comprenant de 1 à 6 atomes de carbone ou l'un des R₁ ou R₂ désigne un atome d'hydrogène, et l'autre des R₁ et R₂ représente un radical alkyle comprenant de 1 à 6 atomes de carbone,
- pyridino,
- amido de formule -NH-COR' ou -CO-NH-R' dans laquelle R' représente un atome d'hydrogène ou un radial alkyle comprenant de 1 à 6 atomes de carbone,
- pyrrolidino choisi de préférence parmi les groupes de formule : 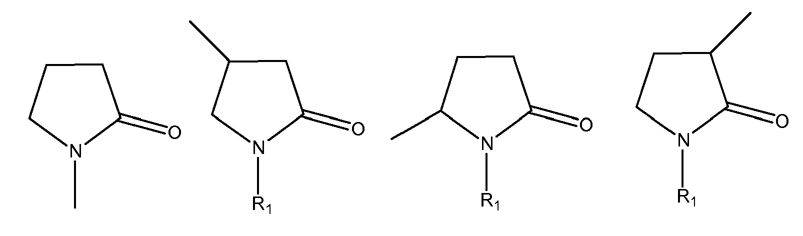 R₁ étant un radial alkyle comprenant de 1 à 6 atomes de carbone,
- carbamoyle de formule -O-CO-NH-R' ou -NH-CO-OR', R' étant tel que défini ci-dessus,
- thiocarbamoyle tel que -O-CS-NH- R' ou -NH-CS-OR', R' étant tel que défini ci-dessus,
- uréyle tel que -NR'-CO-N(R')₂, les groupes R' identiques ou différents étant tels que définis ci-dessus,
- sulfonamido tel que -NR'-S(=O)₂-R', R' répondant à la définition ci-dessus.

De préférence, ces groupes polaires sont présents à une teneur inférieure ou égale à 10 % en poids par rapport au poids de chaque composé X ou Y, de préférence inférieure ou égale à 5 % en poids, par exemple en une teneur allant de 1 à 3 % en poids.

Le ou les groupes polaires peu(ven)t être situé(s) dans la chaîne principale du composé X et/ou Y ou peuvent être pendants à la chaîne principale ou situés aux extrémités de la chaîne principale du composé X et/ou Y.

### 1- Composés X et Y susceptibles de réagir par hydrosilylation

Selon un mode de réalisation, l'invention concerne un kit cosmétique de maquillage ou de soin non thérapeutique de matière(s) kératinique(s) comprenant au moins deux compositions différentes conditionnées séparément, le kit comprenant au moins un composé X, au moins un composé Y, et au moins un catalyseur, avec au moins un des composés X ou Y étant siliconé et lesdits composés X et Y étant susceptibles de réagir ensemble par une réaction d'hydrosilylation en présence d'un catalyseur, lorsqu'ils sont mis en contact les uns avec les autres, ledit kit étant tel que les composés X et Y, et le catalyseur, ne sont pas présents simultanément dans la même composition et qu'au moins l'une des compositions dudit kit est sous la forme d'une émulsion simple ou multiple E/H/E dans laquelle ledit composé X ou Y est présent dans la phase huileuse.

Selon ce mode de réalisation, les composés X et Y sont susceptibles de réagir par hydrosilylation en présence d'un catalyseur, cette réaction pouvant être de manière simplifiée schématisée comme suit : 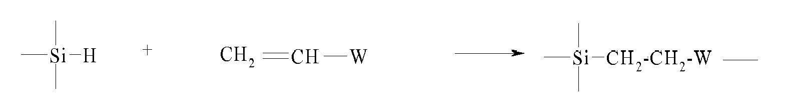 avec W représentant une chaîne carbonée et/ou siliconée contenant un ou plusieurs groupements aliphatiques insaturés.

Dans ce cas, le composé X peut être choisi parmi les composés siliconés comprenant au moins deux groupements aliphatiques insaturés. A titre d'exemple, le composé X peut être un polyorganosiloxane comprenant une chaîne principale siliconée dont les groupements aliphatiques insaturés sont pendants à la chaîne principale (groupe latéral) ou situés aux extrémités de la chaîne principale du composé (groupe terminal). On appellera, dans la suite de la description, ces composés particuliers des polyorganosiloxanes à groupements aliphatiques insaturés.

Selon un mode de réalisation, le composé X et/ou le composé Y est porteur d'au moins un groupe polaire, tel que décrit ci-dessus, susceptible de former au moins une liaison hydrogène avec les matières kératiniques. Ce groupe polaire est avantageusement porté par le composé X qui comprend au moins deux groupements aliphatiques insaturés.

Selon un mode de réalisation, le composé X est choisi parmi les polyorganosiloxanes comprenant au moins deux groupements aliphatique insaturés, par exemple deux ou trois groupements vinyliques ou allyliques, liés chacun à un atome de silicium.

Selon un mode de réalisation avantageux, le composé X est choisi parmi les polyorganosiloxanes comprenant des unités siloxanes de formule : 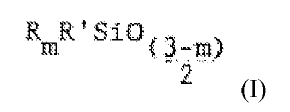 dans laquelle :
- R représente un groupe hydrocarboné monovalent, linéaire ou cyclique, comprenant de 1 à 30 atomes de carbone, de préférence de 1 à 20, et mieux de 1 à 10 atomes de carbone, comme par exemple un radical alkyle à chaîne courte, comprenant par exemple de 1 à 10 atomes de carbone, en particulier un radical méthyle ou encore un groupement phényle, de préférence un radical méthyle,
- m est égal à 1 ou 2 et
- R' représente :
   o un groupement hydrocarboné aliphatique insaturé comprenant de 2 à 10, de préférence de 3 à 5 atomes de carbone comme par exemple un groupe vinyle ou un groupe -R"-CH=CHR''' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène; on peut citer comme groupement R' les groupements vinyle, allyle et leurs mélanges ; ou
   o un groupement hydrocarboné cyclique insaturé comprenant de 5 à 8 atomes de carbone comme par exemple un groupe cyclohexènyle.

De préférence R' est un groupement hydrocarboné aliphatique insaturé, de préférence un groupe vinyle.

Selon un mode de réalisation, R représente un radical alkyle comprenant de 1 à 10 atomes de carbone ou encore un groupement phényle, et de préférence un radical méthyle, et R' est un groupe vinyle.

Selon un mode de réalisation particulier, le polyorganosiloxane comprend également des unités de formule : 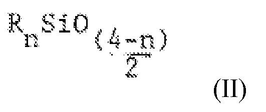 dans laquelle R est un groupe tel que défini plus haut, et n est égal à 1, 2 ou 3.

Selon une variante, le composé X peut être une résine de silicone comprenant au moins deux insaturations éthyléniques, ladite résine étant apte à réagir avec le composé Y par hydrosilylation en présence d'un catalyseur. On peut citer par exemple les résines de type MQ ou MT portant elle-même des extrémités réactives insaturées -CH=CH₂.

Ces résines sont des polymères d'organosiloxanes réticulés.

La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

La lettre M représente l'unité monofonctionelle de formule (CH₃)₃SiO_{1/2}, l'atome de silicium étant relié à un seul atome d'oxygène dans le polymère comprenant cette unité.

La lettre D signifie une unité difonctionnelle (CH₃)₂SiO_{2/2} dans laquelle l'atome de silicium est relié à deux atomes d'oxygène

La lettre T représente une unité trifonctionnelle de formule (CH₃)SiO_{3/2}.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tetrafonctionnelle SiO_{4/2} dans laquelle l'atome de silicium est lié à quatre atomes d'hydrogène eux mêmes liés au reste du polymère. Comme exemples de telles résines, on peut citer les résines de silicone MT telles que les poly(phényl-vinylsilsesquioxane) comme celle commercialisées sous la référence SST-3PV1 par la société Gelest.

De préférence, les composés X comprennent de 0,01 à 1 % en poids de groupes aliphatiques insaturés.

Avantageusement, le composé X est choisi parmi les polyorganopolysiloxanes, notamment ceux comprenant les unités siloxanes (I) et éventuellement (II) décrites précédemment.

Le composé Y comprend de préférence au moins deux groupes Si-H (groupes hydrogénosilanes) libres.

Le composé Y peut être avantageusement choisi parmi les polyorganosiloxanes comprenant au moins une unité alkylhydrogènosiloxane de formule suivante : 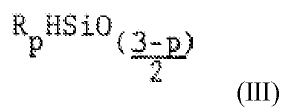 dans laquelle :
R représente un groupe hydrocarboné monovalent, linéaire ou cyclique, comprenant de 1 à 30 atomes de carbone, comme par exemple un radical alkyle ayant de 1 à 30 atomes de carbone, de préférence de 1 à 20 et mieux de 1 à 10 atomes de carbone, en particulier un radical méthyle, ou encore un groupement phényle et p est égal à 1 ou 2. De préférence R est un groupement hydrocarboné, de préférence le méthyle.

Ces composés Y polyorganosiloxanes à unités alkylhydrogènosiloxanes peuvent comprendre en outre des unités de formule : 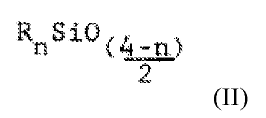 telles que définies plus haut.

Le composé Y peut être une résine de silicone comprenant au moins un motif choisi parmi les motifs M, D, T, Q tels que définis ci-dessus et comprenant au moins un groupe Si-H tel que les poly(méthyl-hydridosilsesquioxane) commercialisées sous la référence SST-3MH1.1 par la société Gelest.

De préférence, ces composés Y polyorganosiloxanes comprennent de 0,5 à 2,5% en poids de groupes Si-H.

Avantageusement, les radicaux R représentent un groupement méthyle dans les formules (I), (II), (III) ci-dessus.

De préférence, ces polyorganosiloxanes Y comprennent des groupes terminaux de formule (CH₃)₃SiO_{1/2}.

Avantageusement, les polyorganosiloxanes Y comprennent au moins deux unités alkylhydrogènosiloxane de formule -(H₃C)(H)SiO- et comprennent éventuellement des unités -(H₃C)₂SiO-.

De tels composés polyorganosiloxanes Y à groupements hydrogénosilane sont décrits par exemple dans le document EP 0465744.

Selon une variante, le composé X est choisi parmi les oligomères ou polymères organiques (par organique, on entend des composés dont la chaîne principale est non siliconée, de préférence des composés ne comprenant pas d'atomes de silicium) ou parmi les polymères ou oligomères hybrides organique/silicone, lesdits oligomères ou polymères portant au moins 2 groupements aliphatiques insaturés réactifs, le composé Y étant choisi parmi les polyorganosiloxanes Y à groupements hydrogénosilane cités ci-dessus.

Selon un mode de réalisation, les composés X organiques ou hybrides organique/silicone portant au moins 2 groupements aliphatiques insaturés réactifs, portent au moins un groupe polaire tel que décrit plus haut.

Le composé X, de nature organique, peut alors être choisi parmi les polymères ou oligomères vinyliques, (méth)acryliques, les polyesters, les polyuréthanes et/ou les polyurées, les polyéthers, les perfluoropolyéthers, les polyoléfines telles que le polybutène, le polyisobutylène, les dendrimères ou les polymères hyper-ramifiés organiques, ou leurs mélanges.

En particulier, le polymère organique ou la partie organique du polymère hybride peut être choisi parmi les polymères suivants :
a) les polyesters à insaturation(s) éthylénique(s) :
   Il s'agit d'un groupe de polymères de type polyester présentant au moins 2 doubles liaisons éthyléniques, réparties de manière aléatoire dans la chaîne principale du polymère. Ces polyesters insaturés sont obtenus par polycondensation d'un mélange :
   - de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP-A-959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
   - de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le le bisphénol A et le bisphénol B, et/ou de dimères diols issus de la réduction des dimères d'acides gras tels que définis précédemment, et
   - d'un ou de plusieurs diacides carboxyliques ou leurs anhydrides comportant au moins une double liaison éthylénique polymérisable et ayant de 3 à 50 atomes de carbone, de préférence de 3 à 20 et mieux de 3 à 10 atomes de carbone, tels que l'acide maléique, l'acide fumarique ou l'acide itaconique.
b) les polyesters à groupes (méth)acrylate latéraux et/ou terminaux :
   Il s'agit d'un groupe de polymères de type polyester obtenus par polycondensation d'un mélange :
   - de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la demande EP-A-959 066 (paragraphe [0021]) commercialisés sous les dénominations Pripol^{®} par la société Unichema ou Empol^{®} par la société Henkel, tous ces diacides devant être exempts de doubles liaisons éthyléniques polymérisables,
   - de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le bisphénol A et le bisphénol B, et
   - d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
   Ces polyesters différent de ceux décrits ci-dessus sous le point a) par le fait que les doubles liaisons éthyléniques ne sont pas situées dans la chaîne principale mais sur des groupes latéraux ou à l'extrémité des chaînes. Ces doubles liaisons éthyléniques sont celles des groupes (méth)acrylate présents dans le polymère.
   De tels polyesters sont commercialisés par exemple par la société UCB sous les dénominations EBECRYL® (EBECRYL® 450 : masse molaire 1600, en moyenne 6 fonctions acrylate par molécule, EBECRYL® 652 : masse molaire 1500, en moyenne 6 fonctions acrylate par molécule, EBECRYL® 800 : masse molaire 780, en moyenne 4 fonctions acrylate par molécule, EBECRYL® 810 : masse molaire 1000, en moyenne 4 fonctions acrylate par molécule, EBECRYL® 50 000 : masse molaire 1500, en moyenne 6 fonctions acrylate par molécule).
c) les polyuréthannes et/ou polyurées à groupes (méth)acrylate, obtenus par polycondensation :
   - de diisocyanates, triisocyanates et/ou polyisocyanates aliphatiques cycloaliphatiques et/ou aromatiques ayant notamment de 4 à 50, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule : 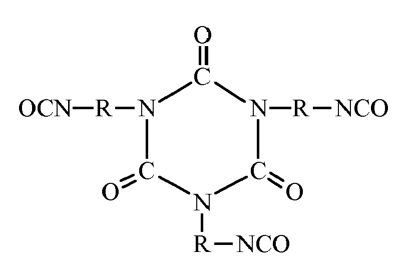 résultant de la trimérisation de 3 molécules de diisocyanates OCN-R-CNO, où R est un radical hydrocarboné linéaire, ramifié ou cyclique comportant de 2 à 30 atomes de carbone;
   - de polyols, notamment de diols, exempts d'insaturations éthyléniques polymérisables, tels que le 1,4-butanediol, l'éthylèneglycol ou le triméthylolpropane, et/ou de polyamines, notamment de diamines, aliphatiques, cycloaliphatiques et/ou aromatiques ayant notamment de 3 à 50 atomes de carbone, telles que l'éthylènediamine ou l'hexaméthylènediamine, et
   - d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.
   De tels polyuréthannes/polyurées à groupes acrylates sont commercialisés par exemple sous la dénomination SR 368 (tris(2-hydroxyéthyl)isocyanurate-triacrylate) ou CRAYNOR® 435 par la société CRAY VALLEY, ou sous la dénomination EBECRYL® par la société UCB (EBECRYL® 210 : masse molaire 1500, 2 fonctions acrylate par molécule, EBECRYL® 230 : masse molaire 5000, 2 fonctions acrylate par molécule, EBECRYL® 270 : masse molaire 1500, 2 fonctions acrylate par molécule, EBECRYL® 8402 : masse molaire 1000, 2 fonctions acrylate par molécule, EBECRYL® 8804 : masse molaire 1300, 2 fonctions acrylate par molécule, EBECRYL® 220 : masse molaire 1000, 6 fonctions acrylate par molécule, EBECRYL® 2220 : masse molaire 1200, 6 fonctions acrylate par molécule, EBECRYL® 1290 : masse molaire 1000, 6 fonctions acrylate par molécule, EBECRYL® 800 : masse molaire 800, 6 fonctions acrylate par molécule).
   On peut également citer les polyuréthannes aliphatiques diacrylate hydrosolubles commercialisés sous les dénominations EBECRYL® 2000, EBECRYL® 2001 et EBECRYL® 2002, et les polyuréthannes diacrylate en dispersion aqueuse commercialisés sous les dénominations commerciales IRR® 390, IRR® 400, IRR® 422 IRR® 424 par la société UCB.
d) les polyéthers à groupes (méth)acrylate obtenus par estérification, par l'acide (méth)acrylique, des groupes hydroxyle terminaux d'homopolymères ou de copolymères d'alkylèneglycols en C₁₋₄, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé.
   Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL® 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL® 160 par la société UCB. Des triacrylates de triméthylolpropane polypropoxylé sont commercialisés par exemple sous les dénominations SR 492 et SR 501 par la société CRAY VALLEY.
e) les époxyacrylates obtenus par réaction entre
   - au moins un diépoxyde choisi par exemple parmi :
      (i) l'éther diglycidylique de bisphénol A,
      (ii) une résine diépoxy résultant de la réaction entre l'éther diglycidylique de bisphénol A et l'épichlorhydrine,
      (iii) une résine époxyester à extrémités α,ω-diépoxy résultant de la condensation d'un diacide carboxylique ayant de 3 à 50 atomes de carbone avec un excès stoechiométrique de (i) et/ou (ii),
      (iv) une résine époxyéther à extrémités α,ω-diépoxy résultant de la condensation d'un diol ayant de 3 à 50 atomes de carbone avec un excès stoechiométrique de (i) et/ou (ii),
      (v) les huiles naturelles ou synthétiques portant au moins 2 groupes époxyde, telles que l'huile de soja époxydée, l'huile de lin époxydée et l'huile de vernonia époxydée,
      (vi) un polycondensat phénol-formaldéhyde (résine Novolac^{®}), dont les extrémités et/ou les groupes latéraux ont été époxydés,
      et
   - un ou plusieurs acides carboxyliques ou polyacides carboxyliques comportant au moins une double liaison éthylénique en α,β du groupe carboxylique comme l'acide (méth)acrylique ou l'acide crotonique ou les esters d'acide (méth)acrylique et d'un diol ou polyol ayant de 2 à 20 atomes de carbone, de préférence de 2 à 6 atomes de carbone tels que le (méth)acrylate de 2-hydroxyéthyle.
   De tels polymères sont commercialisés par exemple sous les dénominations SR 349, SR 601, CD 541, SR 602, SR 9036, SR 348, CD 540, SR 480, CD 9038 par le société CRAY VALLEY, sous les dénominations EBECRYL^{®} 600 et EBECRYL^{®} 609, EBECRYL^{®} 150, EBECRYL^{®} 860, EBECRYL^{®} 3702 par la société UCB et sous les dénominations PHOTOMER^{®} 3005 et PHOTOMER^{®} 3082 par la société HENKEL.
f) les poly(méth)acrylates d'(alkyle en C₁₋₅₀), ledit alkyle étant linéaire, ramifié ou cyclique, comportant au moins deux fonctions à double liaison éthylénique portées par les chaînes hydrocarbonées latérales et/ou terminales.
   De tels copolymères sont commercialisés par exemple sous les dénominations IRR® 375, OTA® 480 et EBECRYL® 2047 par la société UCB.
g) les polyoléfines telles que le polybutène, le polyisobutylène,
h) les perfluoropolyéthers à groupes acrylate obtenus par estérification, par exemple par l'acide (méth)acrylique, de perfluoropolyéthers portant des groupes hydroxyle latéraux et/ou terminaux.
   De tels perfluoropolyéthers α,ω-diols sont décrits notamment dans EP-A-1057849 et sont commercialisés par la société AUSIMONT sous la dénomination FOMBLIN® Z DIOL.
i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique.

Les dendrimères (du grec dendron = arbre) sont des molécules polymères "arborescentes", c'est-à-dire très ramifiées inventées par D. A. Tomalia et son équipe au début des années 90 (Donald A. Tomalia et al., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST® par la société DENDRITECH.

Les polymères hyperramifiés sont des polycondensats, généralement de type polyester, polyamide ou polyéthylèneamine, obtenus à partir de monomères multifonctionnels, qui ont une structure arborescente similaire à celle des dendrimères mais beaucoup moins régulière que celle-ci (voir par exemple WO-A-93/17060 et WO 96/12754).

La société PERSTORP commercialise sous la dénomination BOLTORN® des polyesters hyperramifiés. On trouvera sous la dénomination COMBURST® de la société DENDRITECH des polyéthylèneamines hyperramifiées. Des poly(esteramide) hyperramifiés à extrémités hydroxyle sont commercialisés par la société DSM sous la dénomination HYBRANE®.

Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes. Cette fonctionnalité élevée, le plus souvent supérieure à 5, les rend particulièrement utiles en leur permettant de jouer un rôle de "noeud de réticulation", c'est-à-dire de site de réticulation multiple.

On peut donc utiliser ces polymères dendritiques et hyperramifiés en association avec un ou plusieurs des polymères et/ou oligomères a) à h) ci-dessus.

### 1a - Composés réactifs additionnels

Selon un mode de réalisation, les compositions comprenant le composé X et/ou Y peut comprendre en outre un composé réactif additionnel tels que :
- les particules organiques ou minérales comprenant à leur surface au moins 2 groupements aliphatiques insaturés, on peut citer par exemple les silices traitées en surface par exemple par des composés siliconés à groupements vinyliques tels que par exemple la silice traitée cyclotetraméthyltetravinylsiloxane,
- des composés silazanes tels que l'hexaméthyldisilazane.

### 1b - Catalyseur

La réaction d'hydrosilylation se fait en présence d'un catalyseur qui peut être présent avec l'un ou l'autre des composés X ou Y ou être présent de manière isolée. Par exemple, ce catalyseur peut être présent dans la composition sous une forme encapsulée si les deux composés X et Y, dont il doit provoquer l'interaction, sont présents dans cette même composition sous une forme non encapsulée ou à l'inverse il peut y être présent sous une forme non encapsulée si au moins l'un des composés X et Y est présent dans la composition sous une forme encapsulée. Le catalyseur est de préférence à base de platine ou d'étain.

On peut citer par exemple les catalyseurs à base de platine déposé sur un support de gel de silice ou de poudre de charcoal (charbon), le chlorure de platine, les sels de platine et d'acides chloroplatiniques.

On utilise de préférence les acides chloroplatiniques sous forme hexahydrate ou anhydre, facilement dispersible dans les milieux organosiliconés.

On peut également citer les complexes de platine tels que ceux à base d'acide chloroplatinique hexahydrate et de divinyl tetraméthyldisiloxane.

Le catalyseur peut être présent en une teneur allant de 0,0001 % à 20% en poids par rapport au poids total de la composition le comprenant.

Les composés X et/ou Y peuvent être associés à des inhibiteurs ou retardateurs de polymérisation, et plus particulièrement des inhibiteurs du catalyseur. De façon non limitative, on peut citer les polyméthylvinylsiloxanes cycliques, et en particulier le tetravinyl tétraméthyl cyclotetrasiloxane, les alcools acétyléniques, de préférence volatils, tels que le méthylisobutynol.

La présence de sels ioniques, tels que l'acétate de sodium peut avoir une influence dans la vitesse de polymérisation des composés.

A titre d'exemple d'une combinaison de composés X et Y réagissant par hydrosilylation en présence d'un catalyseur, on peut citer les références suivantes proposée par la société Dow Corning : DC 7-9800 Soft Skin Adhesive Parts A & B, ainsi que la combinaison des mélanges A et B suivants préparés par Dow Corning :

| **MELANGE A :** | | | |
|---|---|---|---|
| **Ingrédient (Nom INCI)** | **N°CAS** | **Teneurs (%)** | **Fonction** |
| Dimethyl Siloxane, Dimethylvinylsiloxy-terminaux | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

| **MELANGE B :** | | | |
|---|---|---|---|
| **Ingrédient (Nom INCI)** | **N°CAS** | **Teneurs (%)** | **Fonction** |
| Dimethyl Siloxane, Dimethylvinylsiloxy-terminaux | 68083-19-2 | 55-95 | Polymère |
| Silica Silylate | 68909-20-6 | 10-40 | Charge |
| Dimethyl, Methylhydrogen Siloxane, trimethylsiloxy-terminaux | 68037-59-2 | 1-10 | Polymère |

De façon avantageuse, les composés X et Y sont choisis parmi les composés siliconés susceptibles de réagir par hydrosilylation en présence d'un catalyseur; en particulier le composé X est choisi parmi les polyorganosiloxanes comprenant des unités de formule (I) décrits ci-dessus et le composé Y est choisi parmi les organosiloxanes comprenant des unités alkylhydrogènosiloxanes de formule (III) décrits ci-dessus.

Selon un mode de réalisation particulier, le composé X est un polydiméthylsiloxane à groupements vinyliques terminaux, et le composé Y est un polyméthylhydrogénosiloxane.

### 2/ Composés X et Y susceptibles de réagir par condensation

Selon un mode de réalisation, l'invention concerne un kit cosmétique de maquillage ou de soin non thérapeutique de matière(s) kératinique(s) comprenant au moins deux compositions différentes conditionnées séparément, le kit comprenant au moins un composé X, au moins un composé Y, et, éventuellement au moins un catalyseur, avec au moins un des composés X ou Y étant siliconé et lesdits composés X et Y étant susceptibles de réagir ensemble par une réaction de condensation, le cas échéant en présence d'un catalyseur, lorsqu'ils sont mis en contact les uns avec les autres, ledit kit étant tel que les composés X et Y et le catalyseur, lorsqu'il est présent, ne sont pas présents simultanément dans la même composition et qu'au moins l'une des compositions dudit kit est sous la forme d'une émulsion simple ou multiple E/H/E dans laquelle ledit composé X ou Y est présent dans la phase huileuse.

Selon ce mode de réalisation, les composés X et Y sont susceptibles de réagir par condensation, soit en présence d'eau (hydrolyse) par réaction de 2 composés porteurs de groupements alcoxysilane, soit par condensation dite « directe » par réaction d'un composé porteur de groupement(s) alcoxysilane et d'un composé porteur de groupement(s) silanol ou par réaction de 2 composés porteurs de groupement(s) silanol.

Lorsque la condensation se fait en présence d'eau, celle-ci peut être en particulier l'humidité ambiante, l'eau résiduelle de la peau, des lèvres, des cils et/ou des ongles, ou l'eau apportée par une source extérieure, par exemple par humidification préalable de la matière kératinique (par exemple par un brumisateur, des larmes naturelles ou artificielles).

Dans ce mode de réaction par condensation, les composés X et Y, identiques ou différents, peuvent donc être choisis parmi les composés siliconés dont la chaîne principale comprend au moins deux groupes alcoxysilane et/ou au moins deux groupes silanol (Si-OH), latéraux et/ou en bout de chaîne.

Selon un mode de réalisation, le composé X et/ou le composé Y est porteur d'au moins un groupe polaire, tel que décrit ci-dessus, susceptible de former au moins une liaison hydrogène avec les matières kératiniques.

Selon un mode de réalisation avantageux, les composés X et/ou Y sont choisis parmi les polyorganosiloxanes comprenant au moins deux groupes alcoxysilane. Par groupe « alcoxysilane », on entend un groupe comprenant au moins une partie -Si-OR, R étant un groupe alkyle comprenant de 1 à 6 atomes de carbone.

Les composés X et Y sont notamment choisis parmi les polyorganosiloxanes comprenant des groupes terminaux alcoxysilanes, plus spécifiquement ceux qui comprennent au moins 2 groupes alcoxysilanes terminaux, de préférence trialcoxysilanes terminaux.

Ces composés X et/ou Y, identiques ou différents, comprennent de préférence de façon majoritaire des unités de formule :

R⁹ₛSiO_{(4-s)/2} (IV)

dans laquelle les groupes R⁹ représentent indépendamment les uns des autres un radical choisi parmi les groupes alkyle comprenant de 1 à 6 atomes de carbone, le phényle, les groupes fluoroalkyle, et s est égal à 0, 1, 2 ou 3. De préférence, les groupes R⁹ représentent indépendamment les uns des autres un groupe alkyle comprenant de 1 à 6 atomes de carbone. Comme groupe alkyle, on peut citer notamment le méthyle, le propyle, le butyle, l'hexyle et leurs mélanges, de préférence le méthyle ou l'éthyle. Comme groupe fluoroalkyle, on peut citer le 3, 3, 3-trifluoropropyle.

Selon un mode de réalisation particulier, les composés X et Y, identiques ou différents, sont des polyorganosiloxanes comprenant des unités de formule :

(R⁹₂SiO₂)_{f} - (V)

dans laquelle R⁹ est tel que décrit ci-dessus, de préférence R⁹ est un radical méthyle, et f est tel que le polymère présente avantageusement une viscosité à 25 °C allant de 0,5 à 3000 Pa.s, de préférence allant de 5 à 150 Pa.s ; par exemple f peut aller de 2 à 5000, de préférence de 3 à 3000, et préférentiellement de 5 à 1000.

Ces composés X et/ou Y polyorganosiloxanes peuvent comprendre au moins 2 groupes trialcoxysilane terminaux par molécule de polymère, lesdits groupes ayant la formule suivante

-ZSiR¹ₓ(OR)₃₋ₓ (VI)

dans laquelle :
les radicaux R représentent indépendamment un groupe méthyle, éthyle, n-propyle, isopropyle, n-butyle, sec-butyle, isobutyle, de préférence un groupe méthyle ou éthyle,
R¹ est un groupe méthyle ou éthyle,
x est égal à 0 ou 1, de préférence x est égal à 0 et
Z est choisi parmi : les groupes hydrocarbonés divalents ne comportant pas d'insaturation éthylénique et comprenant de 1 à 18 atomes de carbone, de préférence de 2 à 18 atomes de carbone (groupes alkylène), les combinaisons de radicaux hydrocarbonés divalents et de segments siloxanes de formule (IX) suivante : 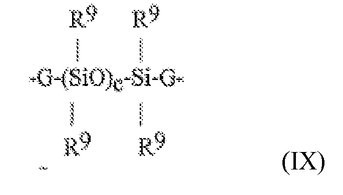 R⁹ étant tel que décrit plus haut, G est un radical hydrocarboné divalent ne comportant pas d'insaturation éthylénique et comprenant de 1 à 18 atomes de carbone, de préférence de 2 à 18 atomes de carbone et c est un entier allant de 1 à 6.
Z et G peuvent être notamment choisis parmi les groupements alkylènes tels que le méthylène, l'éthylène, le propylène, le butylène, le pentylène, l'hexylène, les groupements arylène tels que le phénylène.

De préférence, Z est un groupe alkylène, et mieux éthylène.

Ces polymères peuvent présenter en moyenne au moins 1,2 groupements terminaux ou chaînes terminales trialcoxysilanes par molécule, et de préférence en moyenne au moins 1,5 groupements terminaux trialcoxysilanes par molécule. Ces polymères pouvant présenter au moins 1,2 groupements terminaux trialcoxysilanes par molécule, certains peuvent comprendre d'autre types de groupes terminaux tels que des groupements terminaux de formule CH₂=CH-SiR⁹₂- ou de formule R⁶₃-Si-, dans laquelle R⁹ est tel que défini plus haut et chaque groupe R⁶ est indépendamment choisi parmi les groupes R⁹ ou vinyle. On peut citer comme exemples de tels groupements terminaux les groupes triméthoxysilane, triéthoxysilane, vinyldiméthoxysilane et vinylméthylo xyphénylsilane.

De tels polymères sont notamment décrits dans les documents US 3 175 993, US 4 772 675, US 4 871 827, US 4 888 380, US 4 898 910, US 4 906 719 et US 4 962 174 dont le contenu est incorporé par référence à la présente demande.

On peut citer à titre de composé X et/ou Y en particulier les polyorganosiloxanes choisis parmi les polymères de formule : 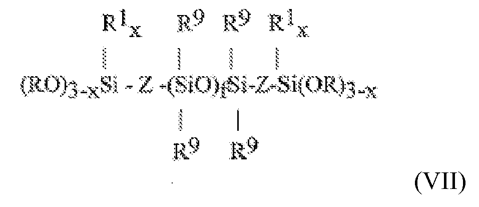 dans laquelle R, R¹, R⁹, Z, x et f sont tels que décrits plus haut.

Les composés X et/ou Y peuvent également comprendre un mélange de polymères de formule (VII) ci-dessus avec des polymères de formule (VIII) suivante : 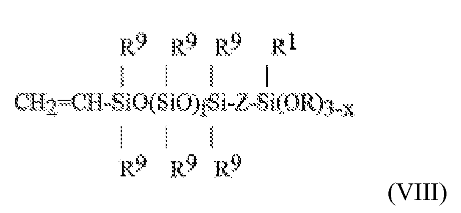 dans laquelle R, R¹, R⁹, Z, x et f sont tels que décrits plus haut.

Lorsque le composé X et/ou Y polyorganosiloxanes à groupe(s) alcoxysilane(s) comprend un tel mélange, les différents polyorganosiloxanes sont présents en des teneurs telles que les chaînes organosilyles terminales représentent moins de 40 %, de préférence moins de 25 % en nombre des chaînes terminales.

Les composés X et/ou Y polyorganosiloxanes particulièrement préférés sont ceux de formule (VII) décrits ci-dessus. De tels composés X et/ou Y sont décrits par exemple dans le document WO 01/96450.

Comme indiqué plus haut, les composés X et Y peuvent être identiques ou différents.

En particulier, les composés X et Y peuvent représenter un mélange de polydiméthylsiloxanes à groupements méthoxysilanes.

Selon une variante, l'un des 2 composés réactifs X ou Y, est de nature siliconée et l'autre est de nature organique. Par exemple le composé X est choisi parmi les oligomères ou polymères organiques ou les oligomères ou polymères hybrides organique/silicone, lesdits polymères ou oligomères comprenant au moins deux groupements alcoxysilanes et Y est choisi parmi les composés siliconés tels que les polyorganosiloxanes décrits ci-dessus. En particulier, les oligomères ou polymères organiques sont choisis parmi les oligomères ou polymères vinyliques, (méth)acryliques, polyesters, polyamides, polyuréthanes et/ou polyurées, polyéthers, polyoléfines, perfluoropolyéthers, dendrimères et polymères hyper-ramifiés organiques, et leurs mélanges.

Selon un mode de réalisation, le composé X de nature organique ou de nature hybride organique/silicone est porteur d'au moins un groupe polaire, tel que décrit ci-dessus, susceptible de former au moins une liaison hydrogène avec la matière kératinique.

Les polymères organiques de nature vinylique ou (méth)acryliques, porteurs de groupes latéraux alcoxysilanes, pourront en particulier être obtenus par copolymérisation d'au moins un monomère organique vinylique ou (méth)acrylique avec un (méth)acryloxypropyltriméthoxysilane, un vinyl triméthoxysilane, un vinyltriéthoxysilane, un allyltriméthoxysilanes etc..

On peut citer par exemple les polymère (méth)acryliques décrits dans le document de KUSABE.M, Pitture e Verniei - European Coating ; 12-B, pages 43-49, 2005, et notamment les polyacrylates à groupes alcoxysilanes référencés MAX de Kaneka ou ceux décrits dans la publication de PROBSTER, M, Adhesion-Kleben & Dichten, 2004, 481 (1-2), pages 12-14.

Les polymères organiques résultant d'une polycondensation ou d'une polyaddition, tels que les polyesters, polyamides, polyuréthanes et/ou polyurées, polyéthers, et porteurs de groupes alcoxysilanes latéraux et/ou terminaux, pourront résulter par exemple de la réaction d'un prépolymère oligomère tel que décrit plus haut avec l'un des co-réactifs silanes suivant porteurs d'au moins un groupe alcoxysilane : aminopropyltriméthoxysilane, aminopropyltriéthoxysilane, aminoéthyl aminopropyl triméthoxysilane, glycidoxypropyltriméthoxysilane, glycidoxypropyltriéthoxysilane, époxycyclohéxyléthyltriméthoxysilane, mercaptopropyltriméthoxysilane.

Des exemples de polyéthers et de polyisobutylènes à groupes alcoxysilanes sont décrits dans la publication de KUSABE.M., Pitture e Verniei - European Coating ; 12-B, pages 43-49, 2005. Comme exemple de polyuréthanes à groupes alcoxysilanes terminaux, on peut citer ceux décrits dans le document PROBSTER, M., Adhesion-Kleben & Dichten, 2004, 481 (1-2), pages 12-14 ou encore ceux décrits dans le document LANDON, S., Pitture e Verniei vol. 73, N° 11, pages 18-24, 1997 ou dans le document HUANG, Mowo, Pitture e Verniei vol. 5, 2000, pages 61-67, on peut notamment citer les polyuréthanes à groupes alcoxysilanes de OSI-WITCO-GE.

A titre de composés X et/ou Y polyorganosiloxane, on peut citer les résines de type MQ ou MT portant elle-même des extrémités alcoxysilanes et/ou silanols comme par exemple les résines poly(isobutylsilsesquioxane) fonctionnalisées par des groupes silanols proposées sous la référence SST-S7C41 (3 groupes Si-OH) par la société Gelest.

### 2a - Composé réactif additionnel

Selon un mode de réalisation, le composé X et/ou Y peut être associé en outre à un composé réactif additionnel comprenant au moins deux groupes alcoxysilane ou silanol. 1.

On peut citer par exemple une ou des particules organiques ou minérales comprenant à leur surface des groupements alcoxysilanes et/ou silanols, par exemple des charges traitées en surface par de tels groupes.

### 2b - Catalyseur

La réaction de condensation peut se faire en présence d'un catalyseur à base de métal qui peut être présent avec l'un ou l'autre des composés X ou Y ou être présent de manière isolée. Par exemple, ce catalyseur peut être présent dans la composition sous une forme encapsulée si les deux composés X et Y, dont il doit provoquer l'interaction, sont présents dans cette même composition sous une forme non encapsulée ou à l'inverse il peut y être présent sous une forme non encapsulée si au moins l'un des composés X et Y est présent dans la composition sous une forme encapsulée. Le catalyseur utile dans ce type de réaction est de préférence un catalyseur à base de titane.

On peut citer notamment les catalyseurs à base de tetraalcoxytitane de formule :

Ti(OR²)_{y}(OR³)_{4-y},

dans laquelle R² est choisi parmi les radicaux alkyle tertiaires tels que le tert butyle, le tert amyle et le 2,4-diméthyl-3-pentyle ; R³ représente un radical alkyle comprenant de 1 à 6 atomes de carbone, de préférence un groupe méthyle, éthyle, n-propyle, isopropyle, n-butyle, sec-butyle, hexyle et y est un nombre allant de 3 à 4, mieux de 3,4 à 4.

Le catalyseur peut être présent en une teneur allant de 0,0001 % à 20% en poids par rapport au poids total de la composition le contenant.

### 2c - Diluant

Les compositions utiles comprenant X et/ou Y peuvent comprendre en outre une huile siliconée volatile (ou diluant) destinée à faire diminuer la viscosité de la composition. Cette huile peut être choisie parmi les silicones linéaires à chaîne courte telles que l'hexaméthyldisiloxane, l'octaméthyltrisiloxane, les silicones cycliques telles que l'octaméthylcyclotetrasiloxane, la decaméthylclopentasiloxane et leurs mélanges.

Cette huile siliconée peut représenter de 5% à 95%, de préférence de 10 à 80% en poids par rapport au poids de chaque composition.

A titre d'exemple d'une combinaison de composés X et Y porteurs de groupements alcoxysilanes et réagissant par condensation, on peut citer la combinaison des mélanges A' et B' suivants préparés par la société Dow Corning :

| **Mélange A' :** | | | |
|---|---|---|---|
| **Ingrédient (Nom INCI)** | **N°CAS** | **Teneurs (%)** | **Fonction** |
| Bis-Trimethoxysiloxyethyl Tetramethyldisilo xyethyl Dimethicone (1) | PMN87176 | 25-45 | Polymère |
| Silica Silylate | 68909-20-6 | 5-20 | Charge |
| Disiloxane | 107-46-0 | 30-70 | Solvant |

| **Mélange B' :** | | | |
|---|---|---|---|
| **Ingrédient (Nom INCI)** | **N°CAS** | **Teneurs (%)** | **Fonction** |
| Disiloxane | 107-46-0 | 80-99 | Solvant |
| Tetra T Butyl Titanate | - | 1-20 | Catalyseur |

Il est à noter que les composés X et Y, identiques, sont réunis dans le mélange A' (cf. (1)).

### 3/ Réticulation en présence de peroxyde :

Selon un mode de réalisation, l'invention concerne un kit cosmétique de maquillage ou de soin non thérapeutique de matière(s) kératinique(s) comprenant au moins deux compositions différentes conditionnées séparément, le kit comprenant au moins un composé X, au moins un composé Y, et au moins un peroxyde, avec au moins un des composés X ou Y étant siliconé et lesdits composés X et Y étant susceptibles de réagir ensemble par une réaction de réticulation en présence d'un peroxyde, lorsqu'ils sont mis en contact les uns avec les autres, ledit kit étant tel que les composés X, Y et le peroxyde ne sont pas présents simultanément dans la même composition et qu'au moins l'une des compositions dudit kit est sous la forme d'une émulsion simple ou multiple E/H/E dans laquelle ledit composé X ou Y est présent dans la phase huileuse.

Cette réaction se fait de préférence par chauffage à une température supérieure ou égale à 50 °C, de préférence supérieure ou égale à 80 °C, allant jusqu'à 120 °C.

Les composés X et Y, identiques ou différents, comprennent dans ce cas au moins deux groupements latéraux -CH₃ et/ou au moins deux chaînes latérales portant un groupement -CH₃.

Les composés X et Y sont de préférence siliconés et peuvent être choisis par exemple parmi les polydiméthylsiloxanes linéaires non volatiles de haut poids moléculaire, ayant un degré de polymérisation supérieur à 6 présentant au moins deux groupements latéraux -CH₃ reliés à l'atome de silicium et/ou au moins deux chaînes latérales portant un groupement -CH₃. On peut citer par exemple les polymères décrits dans le Catalogue « Reactive Silicones » de la société Gelest Inc., Edition 2004, page 6, et notamment les copolymères (aussi appelés gommes) de vinylméthylsiloxane-diméthylsiloxane de poids moléculaires allant de 500 000 à 900 000 et notamment de viscosité supérieure à 2 000 000 cSt.

A titre de peroxydes utilisables dans le cadre de l'invention, on peut citer le peroxyde de benzoyle, le peroxyde de 2,4-dichlorobenzoyle et leurs mélanges.

Selon un mode de réalisation, la réaction d'hydrosilylation en présence d'un catalyseur, ou la réaction de condensation, ou bien encore la réaction de réticulation en présence d'un peroxyde, entre les composés X et Y est accélérée par un apport de chaleur en élevant par exemple la température du système entre 25 °C et 180 °C.

D'une façon générale, quel que soit le type de réaction par laquelle les composés X et Y réagissent ensemble, le pourcentage molaire de X par rapport à l'ensemble des composés X et Y, c'est-à-dire le ratio X/(X+Y) x 100, peut varier de 5 % à 95 %, de préférence de 10 % à 90 %, mieux encore de 20 % à 80 %.

De même, le pourcentage molaire de Y par rapport à l'ensemble des composés X et Y, c'est-à-dire le ratio Y/(X+Y) x 100, peut varier de 5 % à 95 %, de préférence de 10 % à 90 %, mieux encore de 20 % à 80 %.

Le composé X peut présenter une masse moléculaire moyenne en poids (Mw) allant de 150 à 1 000 000, de préférence de 200 à 800 000, de préférence encore de 200 à 250 000.

Le composé Y peut présenter une masse moléculaire moyenne en poids (Mw) allant de 200 à 1 000 000, de préférence de 300 à 800 000, de préférence encore de 500 à 250 000.

Le composé X peut représenter de 0,1 % à 95% en poids par rapport au poids total de la composition le contenant, de préférence de 1% à 90%, et mieux de 5% à 80%.

Le composé Y peut représenter de 0,1 % à 95% en poids par rapport au poids total de la composition le contenant, de préférence de 1% à 90% et mieux de 5% à 80%.

Le ratio entre les composés X et Y peut être varié de manière à moduler la vitesse de réaction et donc la vitesse de formation du film ou encore de manière à adapter les propriétés du film formé (par exemple ses propriétés adhésives) selon l'application recherchée.

En particulier, les composés X et Y peuvent être présents en un ratio X/Y molaire allant de 0,05 à 20 et mieux de 0,1 à 10.

Les composés X et Y peuvent avantageusement être associés à au moins une charge. Ainsi, le kit selon l'invention peut par exemple comprendre dans au moins une des compositions une charge choisie parmi la silice ou la silice traitée en surface.

Comme précisé précédemment, selon un mode de réalisation de l'invention, les composés X et Y peuvent être mis en oeuvre sous la forme d'une unique composition qui contient alors au moins l'un d'entre eux ou le cas échéant le catalyseur ou le peroxyde si nécessaire à leur interaction, sous une forme encapsulée.

Dans le cadre de la présente invention, sont plus particulièrement considérées les formes encapsulées de type coeur/écorce dites encore microcapsules ou nanocapsules dont l'écorce est de nature polymérique et le coeur contient le composé X, le composé Y, l'un de ses composés X et Y étant le cas échéant encapsulé avec le catalyseur ou le peroxyde si nécessaire à l'interaction des deux composés. Dans l'hypothèse où ce catalyseur n'est pas encapsulé avec l'un ou l'autre des composés X ou Y, il est présent dans la composition cosmétique contenant les formes encapsulées.

De nombreuses techniques sont à ce jour disponibles pour préparer ce type de microcapsules ou nanocapsules.

Toutefois, selon un mode préféré, les formes encapsulées considérées selon l'invention sont des nanocapsules et sont obtenues par une technique dite basculement de solvant notamment illustrée dans les documents EP 274 961 et EP 1 552 820.

Plus particulièrement, l'écorce des nanocapsules de composé X ou Y, mises en oeuvre selon l'invention, est de nature polymérique, non réticulée, non hydrosoluble et non soluble dans le coeur des capsules.

D'une manière générale, tous les polymères, d'origine naturelle ou synthétique, solubles dans un solvant non miscible à l'eau et notamment ceux ayant un point de fusion inférieur au point d'ébullition de l'eau à la pression atmosphérique (100 °C), peuvent convenir.

Ces polymères peuvent être biodégradables, comme par exemple les polyesters, ou non.

A titre illustratif des polymères convenant à l'invention, on peut notamment citer :
- les polymères de cyanoacrylate d'alkyle en C₂-C₁₂,
- les polymères formés par les poly-L-lactides, les poly-D/L-lactides, les polyglycolides et les copolymères correspondants,
- les polycaprolactones,
- les polymères de l'acide 3-hydroxy butyrique,
- les copolymères de chlorure de vinyle et d'acétate de vinyle,
- les copolymères d'acide et d'ester méthacrylique, notamment d'acide méthacrylique et d'ester d'acide méthacrylique,
- l'acéto-phtalate de polyvinyle,
- l'acéto-phtalate de cellulose,
- le copolymère polyvinylpyrrolidone-acétate de vinyle,
- les polyéthylènevinylacétates,
- les polyacrylonitriles,
- les polyacrylamides,
- les polyéthylène glycols,
- les poly-(méthacrylate d'hydroxyalkyle en C₁ à C₄)
- les esters de cellulose et d'acide carboxylique en C₁-C₄,
- le polystyrène et les copolymères de styrène et d'anhydride maléique, les copolymères de styrène et d'acide acrylique, les terpolymères séquencés styrène éthylène/butylène-styrène, les terpolymères séquencés styrène-éthylène/propylène-styrène,
- les oligomères styrène alkylalcool,
- les terpolymères d'éthylène, d'acétate de vinyle et d'anhydride maléique,
- les polyamides,
- les polyéthylènes,
- les polypropylènes,
- les organopolysiloxanes dont les polydiméthylsiloxanes,
- les poly (alkylène adipate),
- les polyesters polyol,
- les polymères siliconés de polysilsesquioxane,
- les polyesters dendritiques à fonction hydroxyle terminale,
- les polymères hydrodipersibles mais néanmoins solubles dans des solvants non miscibles à l'eau comme par exemple : les polyesters, poly(ester amides), polyuréthanes et copolymères vinyliques portant des fonctions acide carboxylique et/ou sulfonique et en particulier ceux décrits dans le document FR 2 787 729,
- les copolymères blocs insolubles dans l'eau à température ambiante et solides à température ambiante, ayant au moins un bloc d'un des polymères précédents, et
- leurs mélanges.

Ces polymères ou copolymères peuvent posséder un poids moléculaire moyen en poids compris entre 1000 et 500 000 et en particulier entre 1500 et 100 000.

Conviennent tout particulièrement à l'invention, les poly(alkylène adipate), les organo polysiloxanes, les polycaprolactones, l'acétophtalate de cellulose, l'acétobutyrate de cellulose, les esters de cellulose, le polystyrène, et ses dérivés, et notamment les polycaprolactones.

Bien entendu, l'homme du métier est à même, de par ses connaissances, d'ajuster le poids moléculaire du polymère sélectionné vis-à-vis de sa concentration dans le solvant afin d'avoir une viscosité du mélange compatible avec une émulsification satisfaisante.

En ce qui concerne le coeur lipophile, il peut contenir outre le composé X ou le composé Y, au moins une huile. L'huile peut être choisie parmi les huiles décrites ci-après pour la phase huileuse. L'huile est de préférence une huile siliconée.

Selon une variante de l'invention les formes encapsulées de composé X ou composé Y peuvent être enrobées d'une phase lamellaire.

En ce qui concerne le protocole opératoire pour préparer des nanocapsules convenant à l'invention, l'homme de l'art pourra se reporter notamment à l'enseignement du document EP 1 552 820 cité précédemment. Le choix des tensioactifs requis, ainsi que la mise en oeuvre du procédé, font appel aux connaissances de l'homme de l'art.

### MILIEU PHYSIOLOGIQUEMENT ACCEPTABLE

Comme précisé précédemment, les compositions selon l'invention comprennent un milieu physiologiquement acceptable, c'est à dire un milieu non toxique et susceptible d'être appliqué sur les matières kératiniques d'êtres humains et d'aspect, d'odeur et de toucher agréables.

Les compositions selon l'invention sont généralement sous la forme de compositions de type émulsions directes obtenues par dispersion d'une phase grasse dans une phase aqueuse (H/E), émulsions inverses obtenues par dispersion d'une phase aqueuse dans une phase grasse (E/H) ou émulsions multiples obtenues par dispersion d'une émulsion inverse dans une phase aqueuse (E/H/E). Ces compositions sont préparées selon les méthodes usuelles.

Plus particulièrement, il s'agit d'émulsions directes obtenues par dispersion d'une phase grasse dans une phase aqueuse (H/E).

Selon un mode de réalisation particulier, les émulsions du kit selon l'invention possèdent une phase grasse liquide comprenant au moins une huile.

Dans tous les cas, les composés X et Y sont avantageusement présents dans la phase huileuse.

Comme exemples d'huiles utilisables dans la composition selon l'invention, on peut citer :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïques ou octanoïques ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides capryliques/capriques comme ceux vendus par la société Stearineries Dubois ou ceux vendus sous les dénominations Miglyol 810, 812 et 818 par la société Dynamit Nobel, l'huile de jojoba et l'huile de beurre de karité;
- les esters et les éthers de synthèse, notamment d'acides gras, comme les huiles de formules R₁COOR₂ et R₁OR₂ dans lesquelles R₁ représente le reste d'un acide gras comportant de 8 à 29 atomes de carbone, et R₂ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, l'isohexadecane, l'isododecane, la vaseline, les polydécènes, le polyisobutène hydrogéné tel que l'huile de Parléam^{®};
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polydiméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que le cyclohexasiloxane et le cyclopentasiloxane ; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C₁-C₄-alkyldimethicone et la trifluoropropyldimethicone.

Ces corps gras peuvent être choisis de manière variée par l'homme du métier afin de préparer des compositions ayant les propriétés, par exemple de consistance ou de texture, souhaitées

Les compositions selon l'invention peuvent en outre comprendre une huile volatile.

Par " huile volatile", on entend au sens de l'invention une huile susceptible de s'évaporer au contact des matières kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

On peut citer comme huiles volatiles entre autres, les silicones cycliques ou linéaires renfermant de 2 à 6 atomes de silicium, telles que le cyclohexasiloxane, le dodecamethylpentasiloxane, le decamethyltetrasiloxane, le butyltrisiloxane et l'éthyltrisiloxane. On peut aussi utiliser les hydrocarbures ramifiés tels que par exemple l'isododécane ainsi que les perfluoroalcanes volatiles tels que le dodécafluoropentane et le tétradécafluorohexane, vendus sous les dénominations de "PF 5050^{®}" et "PF 5060^{®}" par la Société 3M et les dérivés de perfluoromorpholine, tels que la 4-trifluorométhyl perfluoromorpholine vendue sous la dénomination "PF 5052^{®}" par la Société 3M.

La quantité de phase huileuse présente dans les compositions selon l'invention peut aller par exemple de 1 % à 90 % en poids, de préférence de 5 % à 70 % en poids par rapport au poids total de la composition considérée.

La teneur en huile volatile présente dans les compositions selon l'invention peut être par exemple inférieure ou égale à 50 % en poids, de préférence inférieure ou égale à 30 % en poids et mieux inférieure ou égale à 10 % en poids par rapport au poids total de la composition considérée.

Selon un mode de réalisation particulier, les compositions selon l'invention sont exemptes d'huile volatile.

Les émulsions contiennent généralement au moins un émulsionnant choisi parmi les émulsionnants amphotères, anioniques, cationiques ou non ioniques, utilisés seuls ou en mélange. Les émulsionnants sont généralement présents dans la composition, en une proportion pouvant aller par exemple de 0,3 à 30 % en poids, et de préférence de 0,5 à 20 % en poids par rapport au poids total de la composition.

Bien entendu, le système émulsionnant est choisi de manière à stabiliser efficacement les émulsions plus particulièrement considérées selon l'invention à savoir de type H/E, E/H, ou H/E/H. Ce choix relève des compétences de l'homme de l'art.

### Tensioactifs

Pour les émulsions H/E, on peut citer par exemple comme émulsionnants, les tensioactifs non ioniques, et notamment les esters de polyols et d'acide gras à chaîne saturée ou insaturée comportant par exemple de 8 à 24 atomes de carbone et mieux de 12 à 22 atomes de carbone, et leurs dérivés oxyalkylénés, c'est-à-dire comportant des unités oxyéthylénées et/ou oxypropylénées, tels les esters de glycéryle et d'acide gras en C₈-C₂₄, et leurs dérivés oxyalkylénés ; les esters de polyéthylène glycol et d'acide gras en C₈-C₂₄, et leurs dérivés oxyalkylénés ; les esters de sorbitol et d'acide gras en C₈-C₂₄, et leurs dérivés oxyalkylénés ; les esters de sucre (sucrose, glucose, alkylglucose) et d'acide gras en C₈-C₂₄, et leurs dérivés oxyalkylénés ; les éthers d'alcools gras ; les éthers de sucre et d'alcools gras en C₈-C₂₄, et leurs mélanges.

Selon un mode de réalisation particulier, les compositions de l'invention contiennent respectivement au moins un tensioactif non ionique, choisi par exemple parmi les esters de polyols et d'acides gras à chaîne saturée ou insaturée et leurs dérivés oxyalkylénés.

Comme ester de glycéryle et d'acide gras, on peut citer notamment le stéarate de glycéryle (mono-, di- et/ou tri-stéarate de glycéryle) (nom CTFA : glyceryl stearate) ou le ricinoléate de glycéryle, et leurs mélanges.

Comme ester de polyéthylène glycol et d'acide gras, on peut citer notamment le stéarate de polyéthylène glycol (mono-, di- et/ou tri-stéarate de polyéthylène glycol), et plus spécialement le monostéarate de polyéthylène glycol 50 OE (nom CTFA : PEG-50 stearate), le monostéarate de polyéthylène glycol 100 OE (nom CTFA : PEG-100 stearate) et leurs mélanges.

On peut aussi utiliser des mélanges de ces tensioactifs, comme par exemple le produit contenant du Glyceryl stearate et du PEG-100 stearate, commercialisé sous la dénomination ARLACEL 165 par la société Uniqema, et le produit contenant du Glyceryl stearate (mono-distéarate de glycéryle) et du stéarate de potassium, commercialisé sous la dénomination TEGIN par la société Goldschmidt (nom CTFA : glyceryl stearate SE).

Comme ester d'acide gras et de glucose ou d'alkylglucose, on peut citer en particulier le palmitate de glucose, les sesquistéarates d'alkylglucose comme le sesquistéarate de méthylglucose, les palmitates d'alkylglucose comme le palmitate de méthylglucose ou d'éthylglucose, les esters gras de méthylglucoside et plus spécialement le diester de méthylglucoside et d'acide oléique (nom CTFA : Methyl glucose dioleate) ; l'ester mixte de méthylglucoside et du mélange acide oléique / acide hydroxystéarique (nom CTFA : Methyl glucose dioleate/hydroxystearate) ; l'ester de méthylglucoside et d'acide isostéarique (nom CTFA : Methyl glucose isostearate) ; l'ester de méthylglucoside et d'acide laurique (nom CTFA : Methyl glucose laurate) ; le mélange de monoester et de diester de méthylglucoside et d'acide isostéarique (nom CTFA : Methyl glucose sesqui-isostearate) ; le mélange de monoester et de diester de méthylglucoside et d'acide stéarique (nom CTFA : Methyl glucose sesquistearate) et en particulier le produit commercialisé sous la dénomination Glucate SS par la société AMERCHOL, et leurs mélanges.

Comme éthers oxyéthylénés d'acide gras et de glucose ou d'alkylglucose, on peut citer par exemple les éthers oxyéthylénés d'acide gras et de méthylglucose, et en particulier l'éther de polyéthylène glycol de diester de méthyl glucose et d'acide stéarique à environ 20 moles d'oxyde d'éthylène (nom CTFA : PEG-20 methyl glucose distearate) tel que le produit commercialisé sous la dénomination Glucam E-20 distearate par la société AMERCHOL ; l'éther de polyéthylène glycol du mélange de monoester et de diester de méthyl glucose et d'acide stéarique à environ 20 moles d'oxyde d'éthylène (nom CTFA : PEG-20 methyl glucose sesquistearate) et en particulier le produit commercialisé sous la dénomination Glucamate SSE-20 par la société AMERCHOL et celui commercialisé sous la dénomination Grillocose PSE-20 par la société GOLDSCHMIDT, et leurs mélanges.

Comme esters de sucrose, on peut citer par exemple le palmito-stéarate de saccharose, le stéarate de saccharose et le mono laurate de saccharose.

Comme éthers d'alcools gras, on peut citer par exemple les éthers de polyéthylène glycol et d'alcool gras comportant de 8 à 30 atomes de carbone, et notamment de 10 à 22 atomes de carbone, tels que les éthers de polyéthylène glycol et d'alcools cétylique, stéarylique, cetéarylique (mélange d'alcools cétylique et stéarylique). On peut citer par exemple les éthers comportant de 1 à 200 et de préférence de 2 à 100 groupes oxyéthylénés, tels que ceux de nom CTFA Ceteareth-20, Ceteareth-30, et leurs mélanges.

Comme éthers de sucre, on peut citer notamment les alkylpolyglucosides, et par exemple le decylglucoside comme le produit commercialisé sous la dénomination MYDOL 10 par la société Kao Chemicals, le produit commercialisé sous la dénomination PLANTAREN 2000 par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Henkel ; le coco-glucoside comme le produit commercialisé sous la dénomination PLANTACARE 818/UP par la société Henkel ; le cétostéaryl glucoside éventuellement en mélange avec l'alcool cétostéarylique, commercialisé par exemple sous la dénomination MONTANOV 68 par la société Seppic, sous la dénomination TEGO-CARE CG90 par la société Goldschmidt et sous la dénomination EMULGADE KE3302 par la société Henkel ; l'arachidyl glucoside, par exemple sous la forme du mélange d'alcools arachidique et béhénique et d'arachidyl glucoside commercialisé sous la dénomination MONTANOV 202 par la société Seppic ; le cocoyléthylglucoside, par exemple sous la forme du mélange (35/65) avec les alcools cétylique et stéarylique, commercialisé sous la dénomination MONTANOV 82 par la société Seppic et leurs mélanges.

Pour les émulsions E/H, on peut citer par exemple comme émulsionnants les dimethicones copolyols tels que le mélange de cyclomethicone et de dimethicone copolyol, vendu sous la dénomination « DC 5225 C » par la société Dow Corning, et les alkyl-dimethicones copolyols tels que le Laurylmethicone copolyol vendu sous la dénomination "Dow Corning 5200 Formulation Aid" par la société Dow Corning et le Cetyl dimethicone copolyol vendu sous la dénomination ABIL EM 90 par la société Goldschmidt, ou le mélange polyglycéryl-4 isostéarate/cétyl diméthicone copolyol/hexyllaurate vendu sous la dénomination ABIL WE 09 par la société Goldschmidt. On peut y ajouter aussi un ou plusieurs co-émulsionnants. De manière avantageuse, le co-émulsionnant peut être choisi dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Comme émulsifiant adapté à l'obtention d'une émulsion E/H, conviennent notamment les tensioactifs polyisobutylene à terminaison succinique estérifiée, tels que ceux commercialisés sous les noms de Lubrizol 5603^{®} et Chemcinnate 2000^{®} par les sociétés Lubrizol et Chemron.

### Polymères amphiphiles

On entend par polymère amphiphile, tous les polymères comportant à la fois une partie hydrophile et une partie hydrophobe et ayant la propriété de former un film séparant deux liquides de polarité différente et permettant ainsi de stabiliser des dispersions liquide - liquide de type direct, inverse ou multiple. Les polymères amphiphiles convenant plus particulièrement réduisent la tension interfaciale eau/huile jusqu'à 10 mN/m, et ce quelle que soit l'huile. Ces polymères sont ioniques (anioniques ou cationiques) ou amphotères. Ils peuvent être hydrosolubles ou hydrodispersibles. On entend par hydrosoluble le fait qu'ils puissent se disperser dans l'eau sous forme de solution moléculaire. On entend par hydrodispersible le fait qu'ils puissent se disperser dans l'eau sous forme particulaire.

Les polymères amphiphiles conformes à l'invention ont généralement un poids moléculaire moyen en nombre allant de 1 000 à 20 000 000 g/mole, de préférence allant de 20 000 à 8 000 000 et plus préférentiellement encore de 100 000 à 700 000 g/mole. Les quantités de polymères amphiphiles utilisées selon l'invention seront choisies de 0,01 à 20 %, de préférence de 0,1 à 10 % et plus préférentiellement encore de 0,2 % à 5 % en poids, par rapport au poids total de la composition le contenant.

On peut utiliser plus particulièrement les copolymères acrylate/C₁₀-C₃₀-alkylacrylate tels que les produits vendus sous les noms PEMULEN TR1, PEMULEN TR2 et CARBOBOL 1382 par la Société GOODRICH, ou bien leurs mélanges. On pourra utiliser aussi les copolymères acrylate/steareth-20 itaconate et copolymères acrylate/ceteth-20 itaconate vendus sous les noms STRUCTURE 2001 et STRUCTURE 3001 par la Société NATIONAL STARCH. A titre de terpolymères pouvant être utilisés, on peut citer le terpolymère acide méthacrylique/acrylate de méthyle/diméthyl m-isopropénylbenzylisocyanate d'alcool béhénylique éthoxylé à 40 OE (c'est-à-dire comportant 40 groupes oxyéthylénés) vendus par la société AMERCHOL sous les dénominations VISCOPHOBE DB 1000 NP3-NP4.

On peut également citer les terpolymères réticulés d'acide méthacrylique, d'acrylate d'éthyle, de polyéthylène glycol (10 OE) éther d'alcool stéarylique (Steareth 10), notamment ceux vendus par la société ALLIED COLLOIDS sous la dénomination SALCARE SC 80.

Les polymères anioniques utilisables selon l'invention sont par exemple les polymères d'acide isophtalique ou d'acide sulfoisophtalique, et en particulier les copolymères de phtalate/sulfoisophtalate/glycol (par exemple diéthylèneglycol/Phtalate/isophtalate/1,4-cyclohexane-diméthanol) vendus sous les dénominations « Eastman AQ polymer » (AQ35S, AQ38S, AQ55S, AQ48 Ultra) par la société Eastman Chemical.

Selon un mode de réalisation particulier, au moins l'une des compositions d'un kit selon l'invention comprend en outre au moins une matière colorante choisie par exemple parmi les pigments, les nacres, les colorants, les matériaux à effet et leurs mélanges.

Ces matières colorantes peuvent être présentes en une teneur allant de 0,01 % à 50 % en poids, de préférence de 0,01 % à 30 % par rapport au poids total de la composition.Les compositions selon l'invention peuvent comprendre une charge notamment en une teneur allant de 0,01 % à 50 % en poids, par rapport au poids total de la composition de préférence allant de 0,01 % à 30 % en poids. Ces charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, le kaolin, la lauroyl-lysine, l'amidon, le nitrure de bore, le sulfate de Baryum, le carbonate de calcium précipité, le carbonate et l'hydro-carbonate de magnésium, l'hydroxyapatite, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition selon l'invention peut en outre contenir divers adjuvants couramment utilisés dans le domaine cosmétique, tels que des séquestrants ; des parfums ; et des épaississants et gélifiants. Les quantités de ces différents adjuvants et leur nature seront choisies de manière à ne pas nuire aux propriétés optiques de la composition.

Selon la fluidité de la composition que l'on souhaite obtenir, on peut incorporer dans la composition, un ou plusieurs gélifiants, notamment hydrophiles, c'est-à-dire solubles ou dispersibles dans l'eau.

Comme gélifiants hydrophiles, on peut citer en particulier les polymères épaississants hydrosolubles ou hydrodispersibles. Ceux-ci peuvent notamment être choisis parmi : les polymères carboxyvinyliques modifiés ou non, tels que les produits commercialisés sous les dénominations Carbopol (nom CTFA : carbomer) par la société Goodrich ; les polyacrylates et polyméthacrylates tels que les produits vendus sous les dénominations de Lubrajel et Norgel par la société GUARDIAN ou sous la dénomination Hispagel par la société HISPANO CHIMICA ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom C.T.F.A. : Polyacrylamide / C13-14 Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les biopolymères polysaccharidiques comme la gomme de xanthane, la gomme de guar, la gomme de caroube, la gomme d'acacia, les scléroglucanes, les dérivés de chitine et de chitosane, les carraghénanes, les gellanes, les alginates, les celluloses telles que la cellulose microcristalline, la carboxyméthylcellulose, l'hydroxyméthylcellullose et l'hydroxypropylcellulose ; et leurs mélanges.

Comme gélifiants lipophiles, on peut citer par exemple les argiles modifiées telles que le silicate de magnésium modifié (bentone gel VS38 de RHEOX), l'hectorite modifiée par le chlorure de distéaryl diméthyl ammonium (nom CTFA : Disteardimonium hectorite) commercialisé sous la dénomination « bentone 38 CE » par la société RHEOX.

Pour une application en particulier au soin ou au maquillage des peaux grasses, la composition selon l'invention peut comprendre au moins un actif choisi parmi : les agents desquamants, les agents anti-séborrhéiques, les agents anti-microbiens, et les agents apaisants.

Pour une application en particulier au soin ou au maquillage des peaux âgées, la composition selon l'invention peut comprendre au moins un actif choisi parmi : les agents desquamants ou hydratants; les agents dépigmentants ou anti-pigmentants ; les agents anti-glycation ; les agents anti-NO ; les agents stimulant la synthèse de macromolécules dermiques ou épidermiques et/ou empêchant leur dégradation ; les agents stimulant la prolifération des fibroblastes ou des kératinocytes et/ou la différenciation des kératinocytes ; les agents myorelaxants ou dermo-décontractants ; les agents anti-radicalaires ou anti-pollution ; les agents tenseurs ; et les agents agissant sur la micro-circulation.

Les produits et compositions selon l'invention peuvent avoir la forme d'un produit de soin ou de maquillage du visage et/ou du corps, et être conditionnés par exemple sous forme de crème en pot ou fluide en tube ou en flacon pompe.

Ils peuvent avantageusement être utilisés pour le soin et/ou le maquillage de la peau, des lèvres, des fibres kératiniques et en particulier des cils et/ou des ongles selon la nature des ingrédients utilisés.

Selon un mode de réalisation, les compositions sont des compositions destinées au maquillage et/ou au soin des lèvres.

En particulier, le kit selon l'invention est destiné à procurer un film de revêtement sur les lèvres, ou sur les ongles.

Selon un autre mode de réalisation, le kit selon l'invention est destiné à procurer un film de revêtement sur des fibres kératiniques.

En particulier, les compositions sont des compositions de revêtement des cils ou des sourcils et plus particulièrement des mascaras.

Selon un autre mode de réalisation, le kit selon l'invention est destiné à procurer un film de revêtement de la peau du corps ou du visage, plus particulièrement des compositions de maquillage ou de soin de la peau du corps ou du visage telles que par exemple des fonds de teint ou des compositions de maquillage du corps.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour chaque composition.

L'invention est illustrée plus en détails par les exemples décrits ci-après. Sauf indication contraire, les quantités indiquées sont exprimées en pourcentage massique.

### EXEMPLES :

Dans les exemples de compositions décrites ci-après, on utilise, à titre de combinaison des composés X et Y, la combinaison des mélanges A et B suivants préparés par la société Dow Corning :

| **MELANGE A :** | | | |
|---|---|---|---|
| **Ingrédient (Nom INCI)** | **N°CAS** | **Teneurs (%)** | **Fonction** |
| Dimethyl Siloxane, Dimethylvinylsiloxy-terminaux | 68083-19-2 | 55-95 | Polymère |
| Silica Silylate | 68909-20-6 | 10-40 | Charge |
| 1,3-Diethenyl-1,1,3,3-Tetramethyldisilo xane complexes | 68478-92-2 | Trace | Catalyseur |
| Tetramethyldivinyldisiloxane | 2627-95-4 | 0.1-1 | Polymère |

| **MELANGE B :** | | | |
|---|---|---|---|
| **Ingrédient (Nom INCI)** | **N°CAS** | **Teneurs (%)** | **Fonction** |
| Dimethyl Siloxane, Dimethylvinylsiloxy-terminaux | 68083-19-2 | 55-95 | Polymère |
| Silica Silylate | 68909-20-6 | 10-40 | Charge |
| Dimethyl, Methylhydrogen Siloxane, trimethylsiloxy-terminaux | 68037-59-2 | 1-10 | Polymère |

### Exemple 1

Composition cosmétique contenant un composé A conforme à l'invention sous la forme d'une émulsion huile dans eau

| Phase A | |
|---|---|
| Mélange de stéarate de glycéryle et de PEG-100 stéarate (ARLACEL^{®} 165 FL de chez Uniqema) | 2,00 g |
| Mélange de tartrate de dialkyle (C₁₄-C₁₅ linéaire), d'alcool cétylstéarylique, d' alcool laurylique oxyéthylene (25 OE) oxypropyléné (25 OP) (Cosmacol PSE de chez Sasol) | 1,50 g |
| Cyclohexasiloxane | 10,00 g |
| Alcool Stearylique | 1,00 g |
| Mélange A | 10,00 g |

| Phase B | |
|---|---|
| Eau | q.s.p. |
| Phenoxyethanol | 1,00 g |
| Sel pentasodique de l'acide éthylène diamine tétramethylène phosphonique | 0,05 g |
| Acide poly acrylamidométhyl propane sulfonique neutralisé partiellement à l'ammoniaque et hautement réticulé (Hostacerin AMPS de chez Clariant) | 0,40 g |
| Gomme xanthane | 0,20 g |

### Exemple 2

Composition cosmétique contenant un composé B complémentaire du composé 1 de l'exemple 1 sous la forme d'une émulsion huile dans eau

| Phase A | |
|---|---|
| Mélange de stéarate de glycéryle et de PEG-100 stéarate (ARLACEL^{®} 165 FL de chez Uniqema) | 2,00 g |
| Mélange de tartrate de dialkyle (C₁₄-C₁₅ linéaire), d'alcool cétylstéarylique, d' alcool laurylique oxyéthylene (25 OE) oxypropyléné (25 OP) (Cosmacol PSE de chez Sasol) | 1,50 g |
| Cyclohexasiloxane | 10,00 g |
| Alcool Stearylique | 1,00 g |
| Mélange B | 10,00 g |

| Phase B | |
|---|---|
| Eau | q.s.p. |
| Phenoxyethanol | 1,00 g |
| Sel pentasodique de l'acide éthylène diamine tétramethylène phosphonique | 0,05 g |
| Acide poly acrylamidométhyl propane sulfonique neutralisé partiellement à l'ammoniaque et hautement réticulé (Hostacerin AMPS de chez Clariant) | 0,40 g |
| Gomme xanthane | 0,20 g |

### Mode opératoire des exemples 1 et 2 :

On chauffe la phase B à environ 75 °C et l'Hostacerine AMPS y est incorporé sous agitation jusqu'à obtention d'un gel homogène.

La phase A est ensuite chauffée à environ 75 °C.

On réalise l'émulsion en incorporant la phase A dans la phase B et l'agitation est maintenue jusqu'à refroidissement complet de la composition à température ambiante.

Mise en évidence des propriétés matifiantes d'un film obtenu à l'issue du mélange des compositions des exemples 1 et 2

Les compositions sous la forme d'émulsion I et II décrites ci-dessus (exemples 1 et 2) sont mélangées en proportion pondérale 50/50 puis le mélange est appliqué sur une carte de contraste (Prufkarte type 24/5 - 250 cm² commercialisée par la société Erichsen) à l'aide d'un tire-film mécanique (épaisseur humide 150 microns). La composition est séchée pendant une nuit à une température de 37 °C. On obtient un film mat et qui ne transfère pas.

Le résultat obtenu est le rapport R entre la réflexion spéculaire et la réflexion diffuse. La valeur de R est d'autant plus faible que l'effet matifiant est important.

Les matités respectives des compositions 1 et II sont également mesurées. Les résultats sont reportés dans le tableau suivant.

| Composition | I | II | Mélange I + II |
|---|---|---|---|
| R | 1,45 ± 0,09 | 1,45 ± 0,10 | 0,66 ± 0,03 |

Ces résultats *in vitro* montrent que les compositions I et II de l'invention, une fois mélangées, étalées et séchées, forment un dépôt doté de bonnes propriétés de matification. La valeur est nettement inférieure à celle attendue avec un mélange des silicones réactives dans deux phases huileuses à même concentration que celle formulées dans la phase huileuse des émulsions conformes à l'invention.

## Revendications

1. Kit cosmétique de maquillage ou de soin non thérapeutique de matière(s) kératinique(s) comprenant au moins deux compositions différentes conditionnées séparément, le kit comprenant au moins un composé X, au moins un composé Y, et au moins un catalyseur avec au moins un des composés X ou Y étant siliconé et lesdits composés X et Y étant susceptibles de réagir ensemble par une réaction d'hydrosilylation en présence d'un catalyseur, lorsqu'ils sont mis en contact les uns avec les autres, ledit kit étant tel que les composés X, Y et le catalyseur ne sont pas présents simultanément dans la même composition et qu'au moins l'une des compositions dudit kit est sous la forme d'une émulsion simple ou multiple E/H/E dans laquelle ledit composé X ou Y est présent dans la phase huileuse.

2. Kit selon la revendication 1 comprenant au moins :
i. une première composition contenant dans un milieu physiologiquement acceptable au moins un composé X et
ii. une seconde composition contenant, dans un milieu physiologiquement acceptable, au moins un composé Y,
avec au moins l'une desdites première et seconde compositions étant sous la forme d'une émulsions simple ou multiple (E/H/E) dans laquelle le composé X ou Y est présent en phase huileuse, l'une au moins desdites compositions contenant, en outre, au moins un catalyseur.

3. Kit selon la revendication 1 ou 2 dans lequel une seule des première et seconde compositions est sous la forme d'une émulsion.

4. Kit selon la revendication 1 ou 2 dans lequel les première et seconde compositions sont sous la forme d'une émulsion.

5. Kit selon la revendication 1 comprenant au moins :
i. une première composition contenant dans un milieu physiologiquement acceptable au moins un composé X et au moins un composé Y, lesdits composés X et Y étant susceptibles de réagir ensemble en présence d'un catalyseur par une réaction d'hydrosilylation, lorsqu'ils sont mis en contact l'un avec l'autre, et
ii. une seconde composition contenant, dans un milieu physiologiquement acceptable, au moins ledit catalyseur nécessaire à l'interaction desdits composés X et Y,
avec ladite première composition étant sous la forme d'une émulsion simple ou multiple (E/H/E) dans laquelle les composés X et Y sont présents en phase huileuse.

6. Kit selon l'une quelconque des revendications précédentes dans lequel la ou les émulsions sont des émulsions directes (H/E).

7. Kit selon l'une quelconque des revendications précédentes, dans lequel les émulsions possèdent une phase grasse liquide comprenant au moins une huile.

8. Kit selon la revendication précédente, dans lequel la ou les huiles sont présentes en une teneur allant de 1 % à 90 % en poids, de préférence de 5 % à 70 % en poids par rapport au poids total de chaque composition.

9. Kit selon l'une quelconque des revendications précédentes, dans lequel la teneur en huile volatile est inférieure ou égale à 50 % en poids, de préférence inférieure ou égale à 30 % en poids et mieux inférieure ou égale à 10 % en poids par rapport au poids total de chaque composition.

10. Kit selon l'une quelconque des revendications précédentes, dans lequel les compositions sont exemptes d'huile volatile.

11. Kit selon l'une quelconque des revendications précédentes, dans lequel les compositions contiennent respectivement au moins un tensioactif non ionique.

12. Kit selon la revendication précédente dans lequel le tensioactif non ionique est choisi parmi les esters de polyols et d'acide gras à chaîne saturée ou insaturée et leurs dérivés oxyalkylénés.

13. Kit selon la revendication précédente dans lequel le tensioactif non ionique est choisi parmi les esters de glycéryle et d'acide gras en C₈-C₂₄, les esters de polyéthylène glycol et d'acide gras en C₈-C₂₄, les esters de sorbitol et d'acide gras en C₈-C₂₄, les esters de sucre et d'acide gras et leurs dérivés oxyalkylénés ; les éthers d'alcools gras ; les éthers de sucre et d'alcools gras et leurs mélanges.

14. Kit selon l'une quelconque des revendications 1 à 13, **caractérisé en ce que** le composé X est choisi parmi les composés siliconés comprenant au moins deux groupements aliphatiques insaturés.

15. Kit selon l'une quelconque des revendications 1 à 14, dans lequel le composé X est un polyorganosiloxane comprenant une chaîne principale siliconée dont les groupements aliphatiques insaturés sont pendants à la chaîne principale (groupe latéral) ou situés aux extrémités de la chaîne principale du composé (groupe terminal).

16. Kit selon la revendication 15, **caractérisé en ce que** le composé X est porteur d'au moins un groupe polaire.

17. Kit selon l'une quelconque des revendications précédentes, **caractérisé en ce que** le composé X est choisi parmi les polyorganosiloxanes comprenant au moins deux groupements aliphatiques insaturés liés chacun à un atome de silicium.

18. Kit selon l'une quelconque des revendications précédentes, **caractérisé en ce que** le composé X est choisi parmi les polyorganosiloxanes comprenant des unités siloxanes de formule : 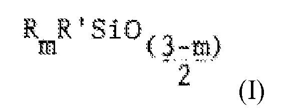 dans laquelle :
- R représente un groupe hydrocarboné monovalent, linéaire ou cyclique, comprenant de 1 à 30 atomes de carbone,
- m est égal à 1 ou 2 et
- R' représente :
• un groupement hydrocarboné aliphatique insaturé comprenant de 2 à 10, de préférence de 3 à 5 atomes de carbone ou
• un groupement hydrocarboné cyclique insaturé comprenant de 5 à 8 atomes de carbone.

19. Kit selon la revendication 18 dans lequel le polyorganosiloxane de formule (I) est tel que R' représente un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène.

20. Kit selon la revendication 18 ou 19, **caractérisé en ce que** R représente un radical alkyle comprenant de 1 à 10 atomes de carbone ou encore un groupement phényle, et de préférence un radical méthyle, et R' est un groupe vinyle.

21. Kit selon l'une quelconque des revendications 15 à 20, **caractérisé en ce que** les polyorganosiloxanes comprennent en outre des unités de formule : 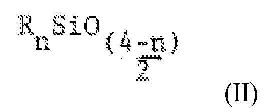 dans laquelle R représente un groupe hydrocarboné monovalent, linéaire ou cyclique, comprenant de 1 à 30 atomes de carbone, et n est égal à 1,2 ou 3.

22. Kit selon l'une quelconque des revendications 1 à 13, **caractérisé en ce que** le composé X est choisi parmi les oligomères ou polymères organiques, les oligomères ou polymères hybrides organique/silicone, lesdits oligomères ou polymères portant au moins 2 groupements aliphatiques insaturés réactifs.

23. Kit selon l'une quelconque des revendications 1 à 22, dans lequel le composé Y comprend au moins deux groupes Si-H libres.

24. Kit selon l'une quelconque des revendications 1 à 23, **caractérisé en ce que** le composé Y est choisi parmi les polyorganosiloxanes comprenant au moins une unité alkylhydrogènosiloxanes de formule suivante : 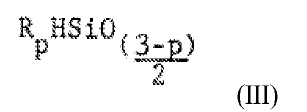 dans laquelle :
R représente un groupe hydrocarboné monovalent, linéaire ou cyclique, comprenant de 1 à 30 atomes de carbone ou un groupe phényle, et p est égal à 1 ou 2.

25. Kit selon la revendication 24, dans lequel le composé Y est tel que les radicaux R représentent un groupement alkyle en C₁-C₁₀, de préférence méthyle.

26. Kit selon l'une des revendications 23 à 25, dans lequel Y est un polyorganosiloxane comprenant au moins deux unités alkylhydrogènosiloxane de formule -(H₃C)(H)Si-O- et comprenant éventuellement des unités -(H₃C)₂SiO-.

27. Kit selon l'une quelconque des revendications 1 à 26, dans lequel le catalyseur est un catalyseur à base de platine ou d'étain.

28. Kit selon la revendication 27, **caractérisé en ce que** le catalyseur est présent en une teneur allant de 0,0001 % à 20 % en poids par rapport au poids total de la composition le comprenant.

29. Kit selon l'une des quelconque des revendications 1 à 23, **caractérisé en ce que** le composé X est un polydiméthylsiloxane à groupements vinyliques terminaux et le composé Y est un polyméthylhydrogénosiloxane.

30. Kit selon l'une quelconque des revendications 1 à 29, dans lequel le composé X est porteur d'au moins un groupe polaire susceptible de former une liaison hydrogène avec les matières kératiniques.

31. Kit selon l'une quelconque des revendications 1 à 30 comprenant dans au moins une des compositions une charge choisie parmi la silice ou la silice traitée en surface.

32. Kit selon l'une quelconque des revendications 1 à 31, **caractérisé en ce que** le composé X présente une masse moléculaire moyenne en poids (Mw) allant de 150 à 1 000 000, de préférence de 200 à 800 000, de préférence encore de 200 à 250 000.

33. Kit selon l'une quelconque des revendications 1 à 32, **caractérisé en ce que** le composé Y présente une masse moléculaire moyenne en poids (Mw) allant de 200 à 1 000 000, de préférence de 300 à 800 000, de préférence encore de 500 à 250 000.

34. Kit selon l'une quelconque des revendications 1 à 33, **caractérisé en ce que** le composé X représente de 0,1 % à 95 % en poids par rapport au poids total de la composition le contenant, de préférence de 1 % à 90 % et mieux de 5 % à 80 %.

35. Kit selon l'une quelconque des revendications 1 à 34, **caractérisé en ce que** le composé Y représente de 0,1 % à 95 % en poids par rapport au poids total de la composition le contenant, de préférence de 1 % à 90 % et mieux de 5 % à 80 %.

36. Kit selon l'une quelconque des revendications 1 à 35, **caractérisé en ce que** les composés X et Y sont présents en un ratio molaire X/Y allant de 0,05 à 20, et mieux de 0,1 à 10.

37. Kit selon l'une quelconque des revendications 1 à 36, dans lequel au moins l'une des compositions comprend en outre au moins une matière colorante.

38. Kit selon l'une quelconque des revendications 1 à 37, dans lequel au moins l'une des compositions comprend au moins une charge.

39. Kit selon l'une quelconque des revendications 1 à 38, dans lequel les compositions sont conditionnées séparément dans un même article de conditionnement.

40. Kit selon l'une quelconque des revendications 1 à 39, destiné à procurer un film de revêtement de la peau du corps ou du visage, plus particulièrement des compositions de maquillage ou de soin de la peau du corps ou du visage en particulier des fonds de teint.

41. Kit selon l'une quelconque des revendications 1 à 39, destiné à procurer un film de revêtement sur les lèvres.

42. Kit selon l'une quelconque des revendications 1 à 39, destiné à procurer un film de revêtement sur les ongles.

43. Kit selon l'une quelconque des revendications 1 à 39, destiné à procurer un film de revêtement sur des fibres kératiniques.

44. Procédé de soin cosmétique et/ou de maquillage de matière(s) kératinique(s) comprenant au moins l'application sous la forme d'au moins une émulsion simple ou multiple (E/H/E) (a) d'un ou plusieurs composés X, (b) d'un ou plusieurs composés Y, avec au moins un des composés X et Y étant siliconé et lesdits composés X et Y étant susceptibles de réagir ensemble par une réaction d'hydrosylilation en présence d'un catalyseur lorsqu'ils sont mis en contact l'un avec l'autre et (c) d'au moins un catalyseur, les applications (a), (b) et (c) pouvant être simultanées ou séquencées selon un ordre indifférent sous réserve qu'il soit propice à l'interaction desdits composés X et Y.

45. Procédé selon la revendication précédente consistant à appliquer sur lesdites matières kératiniques au moins une composition sous la forme d'une émulsion simple ou multiple E/H/E comprenant dans sa phase huileuse au moins un composé X, au moins un composé Y, ladite composition contenant, en outre, au moins un catalyseur.

46. Procédé selon la revendication précédente, dans lequel la composition est obtenue en mélangeant de façon extemporanée au moins une première composition comprenant au moins le composé X et une seconde composition comprenant au moins le composé Y, l'une au moins des première et seconde compositions étant sous la forme d'une émulsion simple ou multiple E/H/E, et comprenant, en outre, au moins un catalyseur.

47. Procédé cosmétique de maquillage et/ou de soin des matières kératiniques notamment de la peau humaine, comprenant l'application sur lesdites matières kératiniques :
i. d'au moins une couche d'une première composition contenant dans un milieu physiologiquement acceptable au moins un composé X ;
ii. d'au moins une couche d'une seconde composition contenant dans un milieu physiologiquement acceptable au moins un composé Y, l'un au moins des composés X et Y étant un composé siliconé, lesdits composés X et Y étant susceptibles de réagir ensemble par une réaction d'hydrosylilation en présence d'un catalyseur, lorsqu'ils sont mis en contact l'un avec l'autre, avec l'une au moins desdites première et seconde compositions étant sous la forme d'une émulsion simple ou multiple E/H/E contenant le composé X ou Y dans sa phase huileuse, et l'une au moins desdites première et seconde compositions contenant en outre au moins un catalyseur.

48. Procédé selon l'une quelconque des revendications 44 à 47 dans lequel la ou les compositions sont telles que définies dans l'une quelconque des revendications 1 à 38.

49. Kit cosmétique de maquillage ou de soin non thérapeutique de matière(s) kératinique(s) comprenant au moins deux compositions différentes conditionnées séparément, le kit comprenant au moins un composé X, au moins un composé Y, et éventuellement au moins un catalyseur avec au moins un des composés X ou Y étant siliconé et lesdits composés X et Y étant susceptibles de réagir ensemble par une réaction de condensation, le cas échéant en présence d'un catalyseur, lorsqu'ils sont mis en contact les uns avec les autres, ledit kit étant tel que les composés X, Y et le catalyseur, lorsqu'il est présent, ne sont pas présents simultanément dans la même composition et qu'au moins l'une des compositions dudit kit est sous la forme d'une émulsion simple ou multiple E/H/E dans laquelle ledit composé X ou Y est présent dans la phase huileuse.
